# EUROPÄISCHE PATENTSCHRIFT

(11) **EP 1 578 391 B1**
(45) Veröffentlichungstag und Bekanntmachung des Hinweises auf die Patenterteilung: **23.04.2014**
(21) Anmeldenummer: 03785925.3
(22) Anmeldetag: 23.12.2003
(51) Int. Cl.: A61Q 17/04, A61Q 19/00, A61K 8/34, A61K 8/81, A61K 8/97, A61Q 19/08

(54) **SELBSTKLEBENDE POLYMERMATRIX MIT EINEM GEHALT AN MEERESALGENEXTRAKT**
SELF-ADHESIVE POLYMER MATRIX CONTAINING A SEAWEED EXTRACT
MATRICE POLYMERE AUTOCOLLANTE CONTENANT UN EXTRAIT DE SEL MARIN

(30) Priorität: 23.12.2002 DE 10260872
(43) Veröffentlichungstag der Anmeldung: 28.09.2005
(73) Patentinhaber: Beiersdorf AG, 20245 Hamburg (DE)
(72) Erfinder: KRUSE, Inge, 20146 Hamburg (DE); WOLBER, Rainer, 22397 Hamburg (DE); WOELLER, Karl-Heinz, 20257 Hamburg (DE)
(86) Internationale Anmeldenummer: PCT/EP2003/014792
(87) Internationale Veröffentlichungsnummer: WO 2004/058211

(56) Entgegenhaltungen:
- EP-A- 1 172 083
- WO-A-01/02478
- WO-A-95/30411
- DE-A- 10 054 479
- FR-A- 2 808 195
- FR-E- 94 977
- US-B1- 6 190 689
- DATABASE CHEMICAL ABSTRACTS [Online] retrieved from STN Database accession no. 136: 90719 XP002275470 & RU 2 158 125 C (V. DROZHZHINA ET AL) 27. Oktober 2000 (2000-10-27)

## Beschreibung

Die vorliegende Erfindung betrifft eine selbstklebende Polymermatrix bestehend aus einem in Wasser gelbildendem Polymer, bevorzugt einem Polyacrylsäurepolymer, Wasser, Meeresalgenextrakt und einem ein- oder mehrwertigen Alkohol. Die Matrix kann mit hydrophilen oder auch hydrophoben Wirkstoffen dotiert werden. Die Wahl des Wirkstoffs oder der Wirkstoffe erfolgt je nach Anwendungsgebiet, das sich aus den jeweiligen Hautbedürfnissen ergibt.

Die Haut ist sich ständig verändernden Umwelteinflüssen ausgesetzt und unterliegt auch im Laufe der Zeit einer Reihe von Veränderungen. So kommt es, wie nachfolgend beschrieben, zu Veränderungen der Barriereeigenschaften, der Hautfaltigkeit- und Elastizität, der Pigmentierung, beispielsweise Altersflecken oder Fehlpigmentierungen wie Melasma u.ä., und insbesondere infolge exogener Einflusse auch zu unterschiedlichen entzündlichen Reaktionen, zu Nachreaktionen der Haut auf die Einwirkung von UV-Strahlung, i.e. Sonnenbrand und/oder die Reizung durch Rasur.

Neben den positiven Auswirkungen des Sonnenlichtes, wie dem allgemeinen Wohlbefinden, der Bildung von Vitamin D3 und der Aknebehandlung, gibt es auch negative Auswirkungen, denen es entgegenzuwirken gilt.

Die schädigende Wirkung des ultravioletten Teils der Sonnenstrahlung auf die Haut ist allgemein bekannt. Während Strahlen mit einer Wellenlänge, die kleiner als 290 nm ist, der sogenannte UVC-Bereich, von der Ozonschicht in der Erdatmosphäre absorbiert werden, verursachen Strahlen im Bereich zwischen 290 nm und 320 nm, dem sogenannten UVB-Bereich, ein Erythem, einen einfachen Sonnenbrand oder sogar mehr oder weniger starke Verbrennungen auf der Haut.

Zum Schutze gegen UVB-Strahlung sind zahlreiche Verbindungen bekannt, bei denen es sich zumeist um Derivate des 3-Benzylidencamphers, der 4-Aminobenzoësäure, der Zimtsäure, der Salicylsäure, des Benzophenons sowie auch des 2-Phenylbenzimidazols handelt.

Etwa 90 % der auf die Erde gelangenden ultravioletten Strahlung besteht aus UV-A-Strahlen mit einer Wellenlänge zwischen 320 nm und 400 nm. Während die UV-B-Strahlung in Abhängigkeit von zahlreichen Faktoren stark variiert, z. B. Jahres- und Tageszeit oder Breitengrad, bleibt die UV-A-Strahlung unabhängig von jahres- und tageszeitlichen oder geographischen Faktoren Tag für Tag relativ konstant. Gleichzeitig dringt der überwiegende Teil der UV-A-Strahlung in die lebende Epidermis ein, während etwa 70 % der UV-B-Strahlen von der Hornschicht zurückgehalten werden.

Zum Schutz gegen die Strahlen des UVA-Bereichs werden daher gewisse Derivate des Dibenzoylmethans verwendet, deren Photostabilität (Int. J. Cosm. Science 10, 53 (1988)), nicht in ausreichendem Maße gegeben ist.

Man hat lange Zeit fälschlicherweise angenommen, dass die langwellige UV-A-Strahlung nur eine vernachlässigbare biologische Wirkung aufweist und dass dementsprechend die UV-B-Strahlen für die meisten Lichtschäden an der menschlichen Haut verantwortlich seien. Inzwischen ist allerdings durch zahlreiche Studien belegt, dass UV-A-Strahlung im Hinblick auf die Auslösung photodynamischer, speziell phototoxischer Reaktionen und chronischer Veränderungen der Haut weitaus gefährlicher als UV-B-Strahlung ist. So ist erwiesen, dass UVA-Strahlung zu einer Schädigung der elastischen und kollagenen Fasern des Bindegewebes führt, was die Haut vorzeitig altern lässt. Hierbei werden Elastizität und Wasserspeichervermögen der Haut reduziert, d.h. die Haut wird weniger geschmeidig und neigt zur Faltenbildung. Diese Art der Faltenbildung wird auch als lichtbedingte Hautalterung bezeichnet. Die auffallend hohe Hautkrebshäufigkeit in Gegenden starker Sonneneinstrahlung zeigt, dass offenbar auch Schädigungen der Erbinformationen in den Zellen durch Sonnenlicht hervorgerufen werden. Auch kann der schädigende Einfluß der UV-B-Strahlung durch UV-A-Strahlung noch verstärkt werden.

Da die Beiträge der verschiedenen Wellenlängebereiche des UV-Lichtes zu lichtbedingten Hautveränderungen nicht vollständig geklärt sind, geht man heute verstärkt davon aus, dass vorbeugender Schutz sowohl gegen UV-A- als auch gegen UV-B-Strahlen, beispielsweise durch Auftrag von Lichtschutzfiltersubstanzen in Form einer kosmetischen oder dermatologischen Formulierung auf die Haut, von grundsätzlicher Wichtigkeit ist. Kosmetische oder dermatologische Mittel sollen, in dünner Schicht auf die Haut aufgetragen, diese vor den negativen Auswirkungen der.Sonnenstrahlung schützen.

Die UV-Strahlung kann, wie zuvor angemerkt, aber auch zu photochemischen Reaktionen führen, wobei dann die photochemischen Reaktionsprodukte in den Hautmetabolismus eingreifen.

Vorwiegend handelt es sich bei solchen photochemischen Reaktionsprodukten um radikalische Verbindungen, z.B. Hydroxylradikale, Auch undefinierte radikalische Photoprodukte, welche in der Haut selbst entstehen, können aufgrund ihrer hohen Reaktivität unkontrollierte Folgereaktionen an den Tag legen. Aber auch Singulettsauerstoff, ein nichtradikalischer angeregter Zustand des Sauerstoffmoleküls kann bei UV-Bestrahlung auftreten, ebenso kurzlebige Epoxide und viele Andere. Singulettsauerstoff beispielsweise zeichnet sich gegenüber dem normalerweise vorliegenden Triplettsauerstoff (radikalischer Grundzustand) durch gesteigerte Reaktivität aus. Allerdings existieren auch angeregte, reaktive (radikalische) Triplettzustände des Sauerstoffmoleküls. Derartige Vorgänge sind über oxidative Schädigung verschiedener Hautstrukturen ganz wesentlich an der lichtbedingten Hautalterung (u.a. Faltenbildung) beteiligt.

Ferner zählt UV-Strahlung zur ionisierenden Strahlung. Es besteht also das Risiko, dass auch ionische Spezies bei UV-Exposition entstehen, welche dann ihrerseits oxidativ in die biochemischen Prozesse einzugreifen vermögen.

Um diesen Reaktionen vorzubeugen, können den kosmetischen bzw. dermatologischen Formulierungen zusätzliche Antioxidantien und/oder Radikalfänger einverleibt werden.

Es ist bereits vorgeschlagen worden, Vitamin E, eine Substanz mit bekannter antioxidativer Wirkung in Lichtschutzformulierungen einzusetzen, dennoch bleibt auch hier die erzielte Wirkung weit hinter der erhofften zurück.

Aufgabe der Erfindung war es daher, kosmetische, dermatologische und pharmazeutische Wirkstoffe in Zubereitungen sowie Lichtschutzformulierungen bereit zu stellen, die zur Prophylaxe und Behandlung lichtempfindlicher Haut, insbesondere Photodermatosen, bevorzugt polymorphe Lichtdermatose dienen.

Weitere Bezeichnungen für die polymorphe Lichtdermatose sind PLD, PLE, Mallorca-Akne und eine Vielzahl von weiteren Bezeichnungen, wie sie in der Literatur (z.B. A. Voelckel et al, Zentralblatt Haut- und Geschlechtskrankheiten (1989), 156, S.2), angegeben sind.

Hauptsächlich werden Antioxidantien als Schutzsubstanzen gegen den Verderb der sie enthaltenden Zubereitungen verwendet. Dennoch ist bekannt, dass auch in der menschlichen und tierischen Haut unerwünschte Oxidationsprozesse auftreten können.
Im Aufsatz "Skin Diseases Associated with Oxidative Injury" in "Oxidative Stress in Dermatology", S. 323 ff. (Marcel Decker Inc., New York, Basel, Hong Kong, Herausgeber: Jürgen Fuchs, Frankfurt, und Lester Packer, Berkeley/Californien), werden oxidative Schäden der Haut und ihre näheren Ursachen aufgeführt.

Auch aus dem Grunde, solchen Reaktionen vorzubeugen, können kosmetischen oder dermatologischen Formulierungen zusätzlich Antioxidantien und/oder Radikalfänger einverleibt werden.

Zwar sind einige Antioxidantien und Radikalfänger bekannt. So ist bereits in den US-Pa-tentschriften 4,144,325 und 4,248,861 sowie aus zahlreichen anderen Dokumenten vorgeschlagen worden, Vitamin E, eine Substanz mit bekannter antioxidativer Wirkung in Lichtschutzformulierungen einzusetzen, dennoch bleibt auch hier die erzielte Wirkung weit hinter der erhofften zurück.

Aufgabe der vorliegenden Erfindung war es somit, Wege zu finden, die die Nachteile des Standes der Technik vermeiden. Insbesondere soll die Wirkung der Behebung der durch Umweltnoxen verursachten Schäden und die Prophylaxe dauerhaft, nachhaltig und ohne das Risiko von Nebenwirkungen sein.

Diesen Übelständen abzuhelfen, war eine Aufgabe der vorliegenden Erfindung.

Ein Sonnenbad wird von den meisten Menschen als angenehm empfunden, die nachteiligen Folgen zunächst nicht beachtet. Allerdings hat sich in den letzten Jahren durchaus ein Bewußtsein über die negativen Auswirkungen einer zu intensiven Sonnenbestrahlung herausgebildet, weshalb mehr und stärker schützende Sonnenschutzmittel angewendet werden.

Der Sonnenbrand bzw. das Lichterythem sind die akuten Erscheinungsformen der Lichteinwirkung. Neben den bereits beschriebenen Wirkungen der UV-Strahlen kommt es in der Nachreaktion der Haut ferner zu einer verminderten Sebumproduktion und einem Austrocknen der Haut. Zur Linderung und zur Pflege der lichtstrapazierten Haut können deshalb spezielle Wirkstoffe eingesetzt werden, wie beispielsweise
- Rückfettungs- und Feuchthaltemittel,
- entzündungslindernde und kühlende Stoffe,
- lokal anaestesierende Stoffe und/oder
- desinfizierende Stoffe, um mögliche Hautinfektionen zu verhindern.

Wie zuvor beschrieben, entwickelt sich nach einer Latenzzeit von 2 bis 3 Stunden eine gegen die unbestrahlte Haut stark abgegrenzte Hautrötung, das Erythema solare, wenn man die Haut zu lange der Sonne oder einer künstlichen Strahlenquelle aussetzt. Bei dem so entstehenden Sonnenbrand unterscheidet man zwischen
■ 1. Grad: Erythem (Rötung, Wärmegefühl)
   klingt nach 2 bis 3 Tagen wieder ab und verschwindet unter gleichzeitig zunehmender Pigmentierung,
■ 2. Grad: Blasenbildung
   auf der Haut bilden sich Blasen mit Brennen und Jucken, die Oberhaut wird flächig abgestoßen
■ 3. Grad: Zellschädiguhg
   es treten tiefgehende Zellschädigungen auf, der Körper reagiert mit Fieber, die Oberhaut wird großflächig abgestoßen.

Der 2. und 3. Grad werden auch als Dermatitis solare bezeichnet.

Die Bildung des Erythems ist abhängig von der Wellenlänge. Der Erythembereich des UV-B liegt zwischen 280 nm und 320 nm. Als ein Maximum der Erythemwirksamkeit des Sonnenlichtes wird der engere Bereich um 308 nm angegeben.

Sogenannte Aftersun-Präparate sind dazu bestimmt, die Haut nach dem Sonnenbad zu kühlen und ihr Feuchthaltevermögen zu verbessern, wobei die Vermittlung des Kühleffektes eine zentrale Rolle spielt. Dieser Kühleffekt wird in der Regel durch hohe Mengen an Ethanol erzielt, welches beim Verteilen der Formulierung auf der Haut spontan verdunstet.

Nachteil dieser Formulierungen des Standes der Technik ist allerdings, dass eine langfristige Kühlung nicht erreichbar ist, da das Ethanol sehr schnell verdunstet und die dadurch entstehende Kühlwirkung dementsprechend nur von kurzer Dauer ist.

Der Stand der Technik kennt weitere Zubereitungen, die - auf der Haut oder Schleimhäuten angewandt - befeuchtend und kühlend wirken sollen. In der Literatur werden beispielsweise ionische Verbindungen, insbesondere Ammoniumsalze, als kühlende Agenzien beschrieben. Als kühlende Zubereitungen werden auch verbreitet isopropanolische Gele mit Campher- und Mentholzusatz angewandt sowie überhaupt häufig etherische Öle, vornehmlich Campher und Menthol, aber auch deren Derivate, z. B. Menthyllactat oder Menthyl-3-hydroxybutyrat, in kühlende Zusammensetzungen eingearbeitet werden.

Menthol, Campher und deren Derivate, aber auch andere etherische Ole erniedrigen die Reizschwelle der neuronalen Kälterezeptoren und rufen so ein Kältegefühl hervor. Häufig bewirken sie aber gleichzeitig eine Durchblutungssteigerung, die im Gegenteil ein Wärmegefühl hervorruft. Die Anwendung dieser Substanzen, namentlich auf gereizter Haut, ist jedenfalls problematisch. Darüber hinaus sind viele dieser Verbindungen schlecht wasserlöslich. Ihre Verwendung ist folglich auf wenige Kosmetika und Dermatika beschränkt.

Aufgabe der vorliegenden Erfindung war es also, kosmetische oder dermatologische Zubereitungen zu finden, die die Nachteile des Standes der Technik nicht aufweisen und die insbesondere lichtstrapazierte Haut langanhaltend pflegen.

Der Wuchs des Barthaares wird beim heranwachsenden Mann durch die gesteigerte Bildung männlicher Hormone während der Pubertät ausgelöst. Hormonelle Störungen bei der Frau können ebenfalls zu einer Form von Bartwuchs führen, die allerdings in aller Regel in seiner Ausprägung deutlich hinter dem des männlichen Bartwuchses zurückbleibt.

Die Rasur des Gesichts oder anderer behaarter Körperteile, wie beispielsweise der Beine, der Achsel oder des Intimbereichs, kann durch mancherlei Zwänge - z. B. religiöser oder kultureller Art - motiviert werden; im einfachsten Fall ist der Haarwuchs für die betreffende Person schlichtweg aus kosmetischen Gründen unerwünscht.

Eine Rasur wird entweder trocken oder naß durchgeführt. Die Entwicklung von neuen mechanischen und elektrischen Naß- und Trockenrasiertechniken ermöglicht heutzutage ein sicheres und gründliches Entfernen des (Bart-) Haares. Bei der Naßrasur sind in der Regel chemische Hilfsmittel - beispielsweise in Form von Rasiergelen, -seifen oder -schäumen - unerläßlich. Diese werden benötigt, um das (Bart-) Haar aufzuweichen und so den zum Durchschneiden erforderlichen Kraftaufwand - und damit das unangenehme Ziehen am Haarschaft - zu minimieren. Das Aufweichen der (Bart-) Haare wird durch Wasseraufnahme erreicht, die durch die Erhöhung des pH-Werts der Haare ermöglicht wird. Naßrasurmittel enthalten deshalb in der Regel Seifen bzw. Fettsäuresalze, deren pH-Wert im Bereich von 8-10 liegt. Produkte für die Naßrasur erzeugen daher ein typisches Hautgefühl, welches nach der Anwendung auftritt. Die Haut fühlt sich trocken und rauh an. Dieses Hautgefühl wird in der kosmetischen Fachwelt auch als "squeakyfeeling" bezeichnet und ist bei Verbrauchern und Verbraucherinnen äußerst unbeliebt.

Auch bei der Trockenrasur sind kosmetische Mittel häufig empfehlenswert, um eine möglichst -dichte Rasur zu bewirken, d. h. das (Bart-) Haar möglichst nahe an der Hautoberfläche abzuschneiden.

Die von der Rasur betroffenen Hautpartien können allerdings nicht nur durch die Rasurhilfsmittel gereizt werden, auch die mechanische Reizung durch das Rasieren an sich stellt eine Belastung der Haut dar, welche ein unangenehmes Hautgefühl, den sog. Rasurbrand", zur Folge haben kann. Eine weitere Aufgabe der vorliegenden Erfindung war es daher, kosmetische oder dermatologische Zubereitungen zu finden, die die Nachreaktionen der Haut auf die (mechanische) Reizung durch das Rasieren besser vermindern.

Die Pigmentierung der menschlichen Haut wird im wesentlichen durch die Gegenwart von Melanin bewirkt. Melanin und dessen Abbauprodukte, Carotin, Durchblutungsgrad sowie die Beschaffenheit und Dicke des Stratum corneum und andere Hautschichten lassen Hautfarbtöne von praktisch weiß, bei verringerter Füllung oder bei Fehlen der Blutgefäße, oder gelblich über hellbraun-rötlich, bläulich bis braun verschiedener Nuancen und schließlich beinahe schwarz erscheinen. Die einzelnen Hautregionen zeigen aufgrund unterschiedlicher Melanin-Mengen unterschiedliche Tiefe der Farbtönung.

Das natürliche Melanin schützt die Haut vor eindringender UV-Strahlung. Die Anzahl der in den Melanocyten produzierten Melanin-Granula entscheidet über Hell- od. Dunkelhäutigkeit. Bei starker Pigmentierung, z.B. bei Farbigen, aber auch bei Hellhäutigen nach einiger UV-Bestrahlung, ist Melanin auch im Stratum spinosum und sogar im Stratum corneum festzustellen. Es schwächt die UV-Strahlung um bis zu ca. 90%, bevor diese das Corium erreicht.

Für die Pigmentierung der Haut verantwortlich sind die Melanozyten, welche in der untersten Schicht der Epidermis, dem Stratum basale, neben den Basalzellen als - je nach Hauttyp entweder vereinzelt oder aber mehr oder weniger gehäuft auftretende pigmentbildende Zellen vorzufinden sind. Melanozyten enthalten als charakteristische Zellorganellen Melanosomen, in denen das Melanin gebildet wird. Unter anderem bei Anregung durch UV-Strahlung wird verstärkt Melanin gebildet. Dieses wird über die lebenden Schichten der Epidermis (Keratinozyten) letztlich in die Hornschicht (Corneozyten) transportiert und ruft eine mehr oder weniger ausgeprägte bräunliche bis braun-schwarze Hautfarbe hervor. Melanin wird als Endstufe eines oxidativen Prozesses gebildet, in welchem Tyrosin unter Mitwirkung der Enzyms Tyrosinase über mehrere Zwischenstufen zu den braun bis braun-schwarzen Eumelaninen, DHICA- und DHI-Melanin, bzw. unter Beteiligung von schwefelhaltigen Verbindungen (Cystein) zum rötlichen Phäomelanin umgewandelt. DHICA- und DHI-Melanin entstehen über die gemeinsamen Zwischenstufen Dopachinon und Dopachrom. Letzteres wird, teilweise unter Beteiligung weiterer Enzyme, entweder in Indol-5,6-Chinon-Carbonsäure oder in Indol-5,6-Chinon umgesetzt, woraus die beiden genannten Eumelanine entstehen. Die Entstehung von Phäomelanin läuft unter anderem über die Zwischenprodukte Dopachinon und Cysteinyldopa.

Neben verschiedenen Funktionen des hauteigenen Melanins, auch "Entgiftung"/Bindung von toxischen Substanzen/Pharmaka usw., ist die Funktion von Melanin als natürlicher UV-Filter zum Schutz vor schädigenden UV-Strahlen sowie die Antioxidansfunktion von Melanin als Schutz vor reaktiven Sauerstoffspezies (oxidativem Stress), die unter anderem durch Sonnenstrahlung auftreten können, für Haut sehr wichtig, u.a. in Bezug auf die Homöostase, Vermeidung von Hautalterung, Vermeidung von Sonnenbrand usw. Somit sollte sich nicht nur ein kosmetischer Nutzen im Sinne einer verstärkte Bräunung durch die gesteigerte Melanin-Synthese in der Haut nach topischer Applikation von die Melanogenese steigernder Verbindungen ergeben sondern auch ein zusätzlicher Schutz durch die verschiedenen Schutzleistungen von Melanin.

Je nach Lichtempfindlichkeit werden in der Regel folgende Hauttypen unterschieden:
Hauttyp I bräunt nie, bekommt immer einen Sonnenbrand.
Hauttyp II bräunt kaum, bekommt leicht einen Sonnenbrand.
Hauttyp III bräunt durchschnittlich gut.
Hauttyp IV bräunt leicht und anhaltend, bekommt fast nie Sonnenbrand.
Hauttyp V dunkle, oft fast schwarze Haut, bekommt nie Sonnenbrand.

Die natürliche Abschirmung der schädlichen UV-Strahlung ist ein handfester Vorteil der natürlichen Hautbräunung. Seit einigen Jahrzehnten gilt darüber hinaus eine "gesunde" Hautfarbe als Zeichen von insbesondere sportlicher Aktivität und wird daher von einer breiten Verbraucherschicht als erstrebenswert erachtet. Vertreter der Hauttypen I und II, die sich einer solchen Hauttönung erfreuen wollen, sind daher ohnehin auf selbstbräunende Präparate angewiesen. Aber auch Vertreter des Hauttyps III, die sich nicht allzu sehr den Risiken des Sonnenbades aussetzen und trotzdem gebräunt aussehen wollen, sind dankbare Zielgruppen für selbstbräunende Zubereitungen.

Die einfachste Art und Weise, seiner Haut einen braunen Farbton zu verleihen, ist das Auftragen entsprechend gefärbter Schminken oder Make-up-Präparate. Allerdings werden selbstverständlich nur solche Körperpartien angefärbt, die von den farbigen Präparaten überdeckt werden. Mit Hilfe abwaschbarer Make-up-Präparate kann eine leichte Hauttönung erzielt werden, z.B. Extrakte aus frischen grünen Walnussschalen, Henna. Ein Nachteil der Schminken ist deshalb die zeitraubende Prozedur des Auftragens. Ferner nachteilig ist, dass sie stark auf Textilien wie Hemdkragen oder Blusen abfärben. Darüber hinaus können die verschiedenen Farbstoffe unterschiedliche allergene Potenz aufweisen und sogar hautirritierend wirken.

Künstliche Hautbräunung lässt sich auf kosmetischem bzw. medizinischem Wege bewirken, wobei im wesentlichen folgende Ansätze eine Rolle spielen:

Durch regelmäßige Einnahme von Carotin-Präparaten wird Carotin wird im Unterhaut-Fettgewebe gespeichert, die Haut färbt sich allmählich orange bis gelbbraun.

Die Anfärbung kann auch auf dem Wege der chemischen Veränderung der Hornschicht der Haut mit sogenannten selbstbräunenden Zubereitungen erfolgen. Wichtigster Wirkstoff ist das Dihydroxyaceton (DHA). Die auf diese Weise erzielte Hautbräunung ist nicht abwaschbar und wird erst mit der normalen Abschuppung der Haut, nach ca. 10-15 Tagen, entfernt. Dihydroxyaceton kann als Ketotriose bezeichnet werden und reagiert als reduzierender Zucker mit den Aminosäuren der Haut bzw. den freien Amino- und IminoGruppen des Keratins über eine Reihe von Zwischenstufen im Sinne einer Maillard-Reaktion zu braungefärbten Stoffen, sogenanten Melanoiden, welche gelegentlich auch Melanoidine genannt werden.
Ein besonderer Nachteil der Bräunung mit Dihydroxyaceton liegt darin, dass die mit ihm gebräunte Haut im Gegensatze zu "sonnengebräunter" Haut nicht gegen Sonnenbrand geschützt ist.
Ein weiterer Nachteil von Dihydroxyaceton besteht darin, dass es, insbesondere unter dem Einfluss ultravioletter Strahlung, wenn auch in meist geringen Mengen Formaldehyd abspaltet. Es war daher ein dringender Bedarf Wege aufzuweisen, auf welchen der Zersetzung von Dihydroxyaceton wirksam begegnet werden kann.

In einer weiteren bevorzugten Ausführungsform betrifft die vorliegende Erfindung kosmetische und dermatologische Zubereitungen zur Prophylaxe und Behandlung kosmetischer oder dermatologischer Hautveränderungen wie z.B. der unerwünschten Pigmentierung, beispielsweise lokale Hyper- und Fehlpigmentierungen, beispielsweise Leberflecken, Sommersprossen, der Inhibierung der natürlichen Pigmentierung, aber auch zur rein kosmetischen Aufhellung größerer, dem individuellen Hauttyp an sich durchaus angemessen pigmentierter Hautflächen.

Probleme mit Hyperpigmentierung der Haut haben vielfältige Ursachen bzw. sind Begleiterscheinungen vieler biologischer Vorgänge, z.B. UV-Strahlung, z.B. Sommersprossen, *Ephelides,* genetische Disposition, Fehlpigmentierung der Haut bei der Wundheilung bzw. -vernarbung oder der Hautalterung, z.B. Altersflecken, *Lentigines seniles.* Altersflecken und unregelmäßige Pigmentierung der menschlichen Haut sind nach heutigem Kenntnisstand insbesondere die Folge von ständiger UV-Sonnen-Bestrahlung und treten, wie der Name Altersfleck schon sagt, im Alter verstärkt auf. Bei Alterflecken kommt es meist zu einer lokalen Vermehrung der Pigment-produzierenden Zellen, der Melanozyten, etwa eine Verdopplung der Melanozyten-Anzahl im Vergleich zur Altersfleck-umgebenden Haut. Hierdurch kommt es auch zu einer verstärkten Pigmentbildung: Der Gehalt an Melanin (Pigment) nimmt im Bereich der Altersflecken zu.

Es sind Wirkstoffe und Zubereitungen bekannt, welche der Hautpigmentierung entgegenwirken. Im praktischen Gebrauch sind im wesentlichen Präparate auf der Grundlage von Hydrochinon, welche aber einesteils erst nach mehrwöchiger Anwendung ihre Wirkung zeigen, deren übertrieben lange Anwendung andererseits aus toxikologischen Gründen bedenklich ist. Auch die Inhibierung der Tyrosinase mit Substanzen wie Kojisäure, Ascorbinsäure und Azelainsäure sowie deren Derivaten ist geläufig, hat aber kosmetische und dermatologische Nachteile.

Diesem Übelstande abzuhelfen, war ebenfalls Aufgabe der vorliegenden Erfindung.

In der Kosmetik ist neben der Hautgesundheit und der Hautpflege auch die Haarpflege ein äußerst intensiv erforschter Bereich.

Haar ist das aus Horn bestehende, fadenförmige, fast universelle, an Handflächen, Fußsohlen, Streckseiten der Zehen-, Fingerendglieder fehlende, Hautanhangsgebilde; unterschieden als Langhaar (die Kopf-, Bart-, Achsel-, Schamhaare = Capilli, Barba, Hirci bzw. Pubes; beim Mann auch Brusthaar), Kurz-, Borstenhaar (Supercilia, Cilia, Vibrissae, Tragi) und Wollhaar (Lanugo, Velushaar). Der Aufbau all dieser Haare ist im groben und ganzen ähnlich: zentral das Haarmark (aus Epithelzellen mit eosinophilen Hornsubstanzkörnchen = Trichohyalin-Granula), umgeben von der Haarrinde (aus verhornten Zellen; enthält Pigmente) und dem Haaroberhäutchen (Cuticula pili; kernlose Epidermisschicht) sowie von Schichten der epithelialen und bindegewebigen Haarscheide.

Das Haar gliedert sich in den aus der Haut ragenden Haarschaft und die in die Unterhaut reichende, schräge Haarwurzel, deren Schichten etwa denen der Oberhaut entsprechen. Das verdickte untere Wurzelende, die Haarzwiebel, sitzt einem in sie hineinragenden, gefäßhaltigen Bindegewebszapfen, der Haarpapille, auf (beide als Haarboden). Die Zwiebel ist in der Anfangs- (= Anagen-) phase, der sich zyklisch wiederholenden Haarbildung zwiebelartig geschichtet infolge ständiger Neubildung von Zellen durch ihre papillennahe Schicht (Matrix), später dann geschlossen, kolbig, ganz verhornt (Kolbenhaar) und wird schließlich, in der End- (= Telogen-) phase, durch ein neues Haar - ausgehend von einer sich neu bildenden Haarpapille - in Richtung Follikelöffnung verdrängt.

Verantwortlich für die persönliche Haarfarbe ist das Melanin. Gebildet wird das Melanin in den Melanozyten, Zellen, die in der Haarzwiebel assoziiert mit den Keratinozyten des Haarmarks vorkommen. Melanozyten enthalten als charakteristische Zellorgänellen Melanosomen, in denen das Melanin gebildet wird. Dieses wird über die langen Dendriten der Melanozyten in die Keratinozyten der präkortikalen Matrix transferiert und ruft die mehr oder weniger ausgeprägte blonde bis braun-schwarze Haarfarbe hervor.

Das Eumelanin ist das Schwarz-Braun-Pigment. Es entscheidend hauptsächlich über die Farbtiefe des Haares. In braunem und schwarzem Haar kommt es in deutlich erkennbaren Körnchen vor.

Das Phaeomelanin ist das Rot-Pigment. Es ist verantwortlich für hellblonde, blonde und rote Haare. Dieses Melanin ist von seiner Struktur her sehr viel feiner und kleiner. Aus den verschiedenen Anteilen der Melanintypen entstehen die verschiedenen Haarfarben:
- Blondes Haar enthält wenig Eumelanin und viel Phaeomelanin.
- Dunkles Haar enthält viel Eumelanin und wenig Phaeomelanin.
- Rotes Haar hat ebenfalls wenig Eumelanin und sehr viel Phaeomelanin.
- Alle dazwischenliegenden Haarschattierungen entstehen aus unterschiedlichen Mischungsverhältnissen der beiden Melanintypen.

Ablaufen kann der Pigmentbildungsprozeß nur, wenn genügend Tyrosinase zur Verfügung steht. Dieses Enzym wird mit zunehmendem Alter seltener gebildet. Das führt dann nach und nach zur grauen Haaren. Der Grund: mit wenig Tyrosinase wird auch immer weniger Tyrosin gebildet. So nimmt auch die Produktion von Melanin ab. Das fehlende Melanin wird durch die Einlagerung von Luftbläschen ersetzt. Die Haare erscheinen grau.

Dieser Prozess ist in der Regel schleichend. Er beginnt an den Schläfen und weitet sich dann auf die gesamte Kopfbehaarung aus. Danach erwischt es den Bart und die Augenbrauen. Zuletzt sind schließlich alle Haare des Körpers grau.

Medizinisch werden graue Haare als Canities bezeichnet. Es gibt verschiedene Möglichkeiten des Ergrauens. Vorzeitiges Ergrauen, ab dem 20 Lebensjahr, nennt sich auch Canities praecox.

Die Canities symptomatica, oder symptomatisches Ergrauen der Haare, kann verschiedene Ursachen haben. Dazu gehören:
- Perniziöse Anämie (Vitamin-B-Mangelanämie),
- schwere endokrinologische Störungen, z. B. bei Schilddrüsenerkrankungen.
- akute, fieberhafte Erkrankungen,
- Arzneimittelnebenwirkungen,
- Kosmetika,
- Metalle.

Die Färbung von Haaren, insbesondere von lebenden menschlichen Haaren, mit Hilfe natürlicher Farbstoffe, wie dies seit dem Altertum insbesondere für den Farbstoff Henna bekannt ist, und die seit Jahren zugunsten synthetischer Farbstoffe in den Hintergrund gedrängt wurden, bildet seit einigen Jahren den Gegenstand eines neuen Interesses. Nachteilig ist der durch Henna entstehende rote Farbton.

Mit zunehmendem Lebensalter nimmt die Melaninproduktion ab, die die Haarfarbe bewirkt: die Haare werden grau bzw. weiß. Es ist ein kosmetischer Wunsch bei einigen Verbrauchern, diesen Prozess umzukehren bzw. langsamer ablaufen zu lassen. Hierzu verwendet die kosmetische Industrie in einigen Ländern Bleiacetat, das giftig ist und daher in der europäischen Kosmetikverordnung verboten ist. Dieses Bleiacetat wird vorzugsweise als Lösung auf die Haare aufgebracht und verbleibt dort längere Zeit, ohne abgewaschen zu werden.

Für das Färben von keratinhaltigen Fasern, z. B. Haaren, Wolle oder Pelzen, kommen im allgemeinen entweder direktziehende Farbstoffe oder Oxidationsfarbstoffe, die durch oxidative Kupplung einer oder mehrerer Entwicklerkomponenten untereinander oder mit einer oder mehreren Kupplerkomponenten entstehen, zur Anwendung. Kuppler- und Entwicklerkomponenten werden auch als Oxidationsfarbstoffvorprodukte bezeichnet.

Als Entwicklerkomponenten werden üblicherweise primäre aromatische Amine mit einer weiteren, in para- oder ortho-Position befindlichen freien oder substituierten Hydroxyoder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminophenol, N,N-Bis-(2-hydroxyethyl)-p-phenylendiamin, 2-(2,5-Diaminophenyl)-ethanol, 2-(2,5-Diaminophenoxy)-ethanol, 1- Phenyl-3-carboxyamido-4-amino-pyrazolon-5, 4-Amino-3-methylphenol, 2-Aminomethyl-4-aminophenol, 2-Hydroxymethyl-4-aminophenol, 2-Hydroxy-4,5,6-triaminopyrimidin, 2,4-Dihydroxy-5,6-diaminopyrimidin und 2,5,6-Triamino-4-hydroxypyrimidin.

Als Kupplerkomponenten werden in der Regel m-Phenylendiaminderivate, Naphthole, Resorcin und Resorcinderivate, Pyrazolone und m-Aminophenole verwendet. Als Kupplersubstanzen eignen sich insbesondere α-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 2,4-diaminophenoxyethanol, 1- Phenyl-3-methylpyrazolon-5, 2,4-Dichlor-3-aminophenol, 1,3-Bis-(2,4-diaminophenoxy)-propan, 2-Chlorresorcin, 4-Chlorresorcin, 2-Chlor-6-methyl-3-aminophenol, 2-Methylresorcin und 5-Methylresorcin.
Bezüglich weiterer üblicher Farbstoffkomponenten wird ausdrücklich auf die Reihe "Dermatology", herausgeben von Ch. Culnan, H. Maibach, Verlag Marcel Dekker Inc., New York, Basel, 1986, Bd. 7, Ch. Zviak, The Science of Hair Care, Kap. 7, Seiten 248-250 (Direktziehende Farbstoffe), und Kap. 8, Seiten 264-267 (Oxidationsfarbstoffe), sowie das "Europäische Inventar der Kosmetikrohstoffe", 1996, herausgegeben von der Europäischen Kommission, erhältlich in Diskettenform vom Bundesverband der deutschen Industrie- und Handelsunternehmen für Arzneimittel, Reformwaren und Körperpflegemittel e.V., Mannheim, Bezug genommen.

Mit Oxidationsfarbstoffen lassen sich zwar intensive Färbungen mit guten Echtheitseigenschaften erzielen, die Entwicklung der Farbe geschieht jedoch im allgemeinen unter dem Einfluss von Oxidationsmitteln wie z. B. H₂O₂, was in einigen Fällen Schädigungen der Faser zur Folge haben kann. Des weiteren können einige Oxidationsfarbstoffvorprodukte bzw. bestimmte Mischungen von Oxidationsfarbstoffvorprodukten bisweilen bei Personen mit empfindlicher Haut sensibilisierend wirken. Direktziehende Farbstoffe werden unter schonenderen Bedingungen appliziert, ihr Nachteil liegt jedoch darin, dass die Färbungen häufig nur über unzureichende Echtheitseigenschaften verfügen.

Aufgabe der vorliegenden Erfindung ist es die selbständige Melaninproduktion der Haare zu verbessern, ohne jedoch auf Färbungsmitteln und insbesondere Oxidationsmittel wie z. B. H₂O₂ angewiesen zu sein. Darüber hinaus dürfen die Mittel kein oder lediglich ein sehr geringes Sensibilisierungspotential aufweisen.

Das heute in der Fachwelt anerkannte Hautmodell von Elias (P. M. Elias, Structure and Function of the Stratum Corneum Permeability Barrier, Drug Dev. Res. 13, 1988, 97-105) beschreibt die Hornschicht als Zwei-Komponenten-System, ähnlich einer Ziegelsteinmauer (Ziegelstein-Mörtel-Modell). In diesem Modell entsprechen die Hornzellen (Korneozyten) den Ziegelsteinen, die komplex zusammengesetzte Lipidmembran in den Interzellularräumen entspricht dem Mörtel. Dieses System stellt im wesentlichen eine physikalische Barriere gegen hydrophile Substanzen dar, kann aber aufgrund seiner engen und mehrschichtigen Struktur gleichermaßen auch von lipophilen Substanzen nur schwer passiert werden. Die besondere Struktur der Hornschicht schützt einerseits die Haut und stabilisiert andererseits ihre eigene Flexibilität durch Bindung einer definierten Wassermenge.

Auch mechanische Belastungen, wie beispielsweise Druck-, Stoß- oder Scherkräfte, können in erstaunlichem Maße durch die Hornschicht allein oder im Verbund mit den tieferen Hautschichten abgefangen werden. Größere Druck-, Dreh- oder Scherkräfte werden über die Verzahnung der Epidermis mit dem Corium an tiefere Hautschichten weitergegeben.

Die Regulation des Wasser- und Feuchtigkeitsgehaltes ist eine der wichtigsten Funktionen der epidermalen Lipidmembran. Allerdings hat sie nicht nur eine Barrierewirkung gegen externe chemische und physikalische Einflüsse, sondern trägt auch zum Zusammenhalt der Hornschicht bei.

Die Lipide der Hornschicht bestehen im wesentlichen aus Ceramiden, freien Fettsäuren, Cholesterin sowie Cholesterinsulfat und sind über die gesamte Hornschicht verteilt. Die Zusammensetzung dieser Lipide ist für die intakte Funktion der epidermalen Barriere und damit für die Wasserundurchlässigkeit der Haut von entscheidender Bedeutung.

Bereits bei einer Reinigung der Haut mit Hilfe eines einfachen Wasserbads - ohne Zusatz von Tensiden - kommt es zunächst zu einer Quellung der Hornschicht der Haut. Der Grad dieser Quellung hängt u. a. von der Dauer des Bads und dessen Temperatur ab. Gleichzeitig werden wasserlösliche Stoffe ab- bzw. ausgewaschen, wie z. B. wasserlösliche Schmutzbestandteile, aber auch hauteigene Stoffe, die für das Wasserbindungsvermögen der Hornschicht verantwortlich sind. Durch hauteigene oberflächenaktive Stoffe werden außerdem auch Hautfette in gewissem Ausmaß gelöst und ausgewaschen. Dies bedingt nach anfänglicher Quellung eine nachfolgende Austrocknung der Haut, die durch waschaktive Zusätze noch deutlich verstärkt werden kann.

Bei gesunder Haut sind diese Vorgänge im allgemeinen belanglos, da die Schutzmechanismen der Haut solche leichten Störungen der oberen Hautschichten ohne weiteres kompensieren können. Aber bereits im Fall nichtpathologischer Abweichungen vom Normalstatus, z. B. durch umweltbedingte Abnutzungsschäden bzw. Irritationen, Lichtschäden, Altershaut usw., ist der Schutzmechanismus an der Hautoberfläche gestört.

Bei alter Haut beispielsweise erfolgt die regenerative Erneuerung verlangsamt, wobei insbesondere das Wasserbindungsvermögen der Hornschicht nachläßt. Sie wird deshalb inflexibel, trocken und rissig ("physiologisch" trockene Haut). Ein Barriereschaden ist die Folge. Die Haut wird anfällig für negative Umwelteinflüsse wie die Invasion von Mikroorganismen, Toxinen und Allergenen. Als Folge kann es sogar zu toxischen oder allergischen Hautreaktionen kommen.

Bei pathologisch trockener und empfindlicher Haut liegt ein Barriereschaden a priori vor. Epidermale Interzellularlipide werden fehlerhaft oder in ungenügender Menge bzw. Zusammensetzung gebildet. Die Konsequenz ist eine erhöhte Durchlässigkeit der Hornschicht und ein unzureichender Schutz der Haut vor Verlust an hygroskopischen Substanzen und Wasser.

Die Barrierewirkung der Haut kann über die Bestimmung des transepidermalen Wasserverlustes (TEWL - transepidermal water loss) quantifiziert werden. Dabei handelt es sich um die Abdunstung von Wasser aus dem Körperinneren ohne Einbeziehung des Wasserverlustes beim Schwitzen. Die Bestimmung des TEWL-Wertes hat sich als außerordentlich informativ erwiesen und kann zur Diagnose rissiger oder schrundiger Haut, zur Bestimmung der Verträglichkeit chemisch verschiedenartig aufgebauter Tenside und dergleichen mehr herangezogen werden.

Für die Schönheit und Gepflegtheit der Haut ist der Wasseranteil in der obersten Hautschicht von größter Bedeutung. Man kann ihn in einem begrenzten Umfang durch Einbringen von Feuchtigkeitsregulatoren günstig beeinflussen.

Anionische Tenside, welche im allgemeinen Bestandteile von Reinigungszubereitungen sind, können den pH-Wert in der Hornschicht langanhaltend erhöhen, was regenerative Prozesse, die der Wiederherstellung und Erneuerung der Barrierefunktion der Haut dienen, stark behindert. In diesem Fall stellt sich in der Hornschicht zwischen Regeneration und dem Verlust essentieller Substanzen durch regelmäßige Extraktion ein neuer, häufig sehr ungünstiger Gleichgewichtszustand ein, der das äußere Erscheinungsbild der Haut und die physiologische Funktionweise der Hornschicht entscheidend beeinträchtigt.

Unter kosmetischer Hautpflege ist in erster Linie zu verstehen, dass die natürliche Funktion der Haut als Barriere gegen Umwelteinflüsse, z.B. Schmutz, Chemikalien, Mikroorganismen, und gegen den Verlust von körpereigenen Stoffen, z. B. Wasser, natürliche Fette, Elektrolyte, gestärkt oder wiederhergestellt wird.

Wird diese Funktion gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliche Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Die chronologische Hautalterung wird z.B. durch endogene, genetisch determinierte Faktoren verursacht. In Epidermis und Dermis kommt es alterungsbedingt z.B. zu folgenden Strukturschäden und Funktionsstörungen, die auch unter den Begriff "Senile Xerosis" fallen können:
a) Trockenheit, Rauhigkeit und Ausbildung von Trockenheitsfältchen,
b) Juckreiz und
c) verminderte Rückfettung durch Talgdrüsen, z.B. nach Waschen.
   Exogene Faktoren, wie UV-Licht und chemische Noxen, können kumulativ wirksam sein.
   In Epidermis und Dermis kommt es insbesondere durch exogene Faktoren z.B. zu folgenden Strukturschäden- und Funktionsstörungen in der Haut:
d) vergrößerte Anfälligkeit gegenüber mechanischem Stress, z.B. Rissigkeit.

Produkte zur Pflege sensibler, juckender und oder trockener Haut bzw. Produkte zur Behandlung von oder Prophylaxe vor DNS-Schädigungen sind an sich bekannt. Allerdings ist deren Wirksamkeit begrenzt.
Insbesondere betrifft die vorliegende Erfindung kosmetische Zubereitungen mit einem zusätzlichen, wirksamen Schutz vor schädlichen Oxidationsprozessen in der Haut, aber auch zum zusätzlichen Schutze kosmetischer Zubereitungen selbst bzw. zum zusätzlichen Schutze der Bestandteile kosmetischer Zubereitungen vor schädlichen Oxidationsprozessen.

Es war Aufgabe der vorliegenden Erfindung, kosmetische und dermatologische Zubereitungen auf Basis einer Gelmatrix, insbesondere unter Einbeziehung geeigneter Wirkstoffe, zu schaffen, die den oben beschriebenen unerwünschten Hauterscheinungen wie
- der Trockenheit, Rauhigkeit, Rissigkeit und verminderten Rückfettung der Haut,
- den Nachwirkungen von exogenen Einwirkungen wie UV-Licht und Rasur, insbesondere dem Sonnenbrand und dem Rasurbrand,
- sonstigen irritativen und entzündlichen Hautreaktionen, u.a. Hautjucken,
- dem Wachstum der Haare kosmetisch erwünscht, z.B. beim Damenbart,
- Altershauterscheinungen wie mangelnder Elastizität, zunehmender Faltigkeit und Altersflecken
   entgegenwirken und die Hautphysiologie hinsichtlich
- der Barriereeigenschaften,
- der Erhaltung der Hauthomoeostase,
- das Wachstum der Haare kosmetisch erwünscht stärken sowie eine kosmetisch erwünschte
- Modulation der Pigmentierung von Haut und Haaren
   ermöglichen.

Bei topischer Anwendung der erfindungsgemäßen kosmetischen oder dermatologischen Zubereitungen mit einem wirksamen Gehalt an verwendeten Wirkstoffen ist in überraschender Weise eine wirksame Behandlung, aber auch eine Prophylaxe bei
- einer verminderten Hauthydratation bzw. eines verminderten Feuchtigkeitsgehaltes der Haut
- Altershautfalten
- Lichtgealterter Haut
- Fettiger und unreiner Haut
- verringerter Hautelastizität
- Altersflecken und sonstigen Fehlpigmentierungen der Haut, z.B. Melasma,
- Nachreaktionen der Haut auf UV-Licht (Sonnenbrand)
- Nachreaktionen der Haut auf die Rasur (Rasurbrand)
- Dysfunktion der Stoffwechselhomeostase der Haut
- einer verminderten Zell-Zell-Kommunikation
- einer verminderten DNS-Synthese und/oder einer verminderten DNS-Reparatur
- einer Aktivierung von Metalloproteinasen und/oder Proteasen
- einer Aktivierung der Cyclooxigenasen und Lipoxygenasen der Haut
- Veränderungen der normalen Hyaluronsäure- und Glucosaminoglycan-Homeostase
- Abweichungen von den normalen posttranslationalen Modifikationen von Bindegewebsproteinen, Glycosaminoglycanen und anderen Strukturbestandteilen
- von Störungen des Ceramid-, Lipid- und Energiestoffwechsels der Haut
- von Störungen des Melanin-Stoffwechsels und der Melanosomen-Prozessierung der Haut
- von defizitären, sensitiven oder hypoaktiven Hautzuständen oder defizitären, sensitiven oder hypoaktiven Zustände von Hautanhangsgebilden
- Veränderungen des transepidermalen Wasserverlustes
- Veränderungen des natural moisturizing factor Gehaltes
- Veränderungen der normalen Lipidperoxidation
- bei entzündlichen Erscheinungen und oder Juckreiz
- bei Schuppenbildung im Haarbereich
- bei vermindertem wie auch bei unerwünschtem Haarwuchs
- von Störungen der Barrierefunktion möglich. Ferner zeigte sich, dass bei Anwendung einer erfindungsgemäßen Zubereitung das allgemeine Frischegefühl der Haut gesteigert werden kann.

Der Wirkmechanismus von Pflastern bzw. kosmetischen Matrices zur Verabreichung kosmetischen Substanzen in und an die Haut unterliegt einem analogen Funktionsprinzip wie Transdermale Therapeutische Systeme (TTS). Die Begriffe Pflaster, kosmetische/dermatologische Matrices und kosmetische/dermatologische Pads werden im Nachfolgenden teilweise synonym benutzt.
Transdermale Therapeutische Systeme zur Abgabe von Wirkstoffen in bzw. durch die Haut sind seit langer Zeit bekannt und stellen pflasterartige, insbesondere arzneistoffdotierte Systeme dar.

Die topische Applikation von kosmetischen und dermatologischen Wirkstoffen über Pflastersysteme bzw. kosmetische Matrices bietet zwei Hauptvorteile:
- Erstens wird durch diese Darreichungsform eine Freisetzungskinetik des Wirkstoffes erster Ordnung realisiert, wodurch über einen sehr langen Zeitraum ein konstanter Wirkstoffspiegel in der Haut aufrechterhalten werden kann.
- Zweitens werden kann über geeignete Systeme eine zusätzliche intensive Pflege der Haut herbeigeführt werden.

Die zeitabhängige Freisetzung des kosmetischen Wirkstoffs aus einem TTS erfolgt in Abhängigkeit ihres Verteilungskoeffizienten TTS/Haut und ihrer Diffusion im Bereich des TTS und der Haut.
Beide Faktoren werden durch die Zusammensetzung der Matrix bestimmt, wodurch die pro Zeiteinheit freigesetzte Menge und die Dauer der Wirksamkeit direkt beeinflusst werden können. Üblicherweise werden hierfür Hydrokolloide, Lösungsvermittler und Enhancer eingesetzt, welche eine verbesserte Löslichkeit und Diffusion sowie einen schnelleren Übergang der Substanz von TTS in die Haut ermöglichen.
Im Idealfall wird eine Freisetzungskinetik erster Ordnung erreicht, was eine Freisetzung gleicher Mengen pro Zeiteinheit ermöglicht.

Eine in der Fachliteratur gut beschriebene Ausführungsform solcher transdermalen Systeme stellen Matrixsysteme öder monolithische Systeme dar, in denen der kosmetische Wirkstoff direkt in den druckempfindlichen Haftklebstoff eingearbeitet wird. Eine solche haftklebrige, wirkstoffhaltige Matrix ist üblicherweise im anwendungsfertigen Produkt auf der einen Seite mit einem für den Wirkstoff undurchlässigen Träger ausgestattet, auf der gegenüberliegenden Seite befindet sich eine mit einer Trennschicht ausgestatten Trägerfolie, die vor der Applikation auf die Haut entfernt wird (kleben&dichten, Nr.42, 1998, S. 26 bis 30).

Die vorgenannten Eigenschaften eines TTS vermeiden eine häufig zu wiederholende Applikation und Belastung der Haut mit hohen Konzentrationen an Wirkstoffen und verringern damit die Reizung der Haut, welche bei wiederholter Applikation von flüssigen und halbfesten Applikationsformen unumgänglich ist.

Zusammengefasst liegen die Vorteile der TTS in einer deutlich verbesserten Compliance der Anwender, was auf die einfache und schnelle Applikation sowie die lange Wirksamkeit von Transdermalen Therapeutischen Systemen zurückzuführen ist.
Eine Grundanforderung an ein TTS ist einerseits ein gutes Haftvermögen auf Haut, das über den gesamten Zeitraum der beabsichtigten Wirkstoffdosierung aufrechterhalten bleiben muss, und andererseits eine rückstandsfreie Entfernbarkeit des TTS. Auch ein schmerzhaftes Wiederablösen des wirkstoffhaltigen Pflasters nach längerer Tragezeit wird häufig beobachtet. Neben Klebmassen, die in Lösung auf den Träger beschichtet werden, kommen u.a. auch lösungsmittelfreie Systeme, wie Heißschmelzklebmassen zum Einsatz. Diese zeichnen sich dadurch aus, dass bei der Beschichtung auf die Verwendung von organischen Lösungs- und Dispergiermittel verzichtet werden kann. Heißschmelzklebmassen werden durch Erwärmen in eine flüssige Form überführt und so als Schmelze auf den jeweiligen Pflasterträger aufgebracht. Neben technischen Gesichtspunkten, wie Lösungsmittelaufbereitung, Ausführungen der Anlagen mit Explosionsschutz und Umweltschutzauflagen, spielen auch medizinische Gründe für die Wahl von lösungsmittelfreien Klebstoffen eine Rolle. Transdermale therapeutische Systeme werden in der Regel auf gesunder, intakter Haut appliziert.

Selbstklebende Matrixsysteme zur Verabreichung von kosmetischen Wirkstoffen gehören in Asien, insbesondere in Japan, zu den traditionellen Anwendungen und werden im japanischen Arzneibuch unter dem Begriff "Cataplasma" definiert. Cataplasmen werden danach gewöhnlich durch Mischen von Glycerin, Wasser oder anderer geeigneter flüssiger Substanzen mit fein pulverisierten Wirkstoffen unter Zusatz essentiellen Ölen zubereitet.

Glycerin fungiert hierbei als Feuchthaltemittel, um ein vorzeitiges Austrocknen bei Anwendung der Cataplasmen zu verhindern.

Während in den traditionellen asiatischen Zubereitungen natürliche Verdickungsmittel wie Tonerde etc. zur Anwendung kommen, werden in den letzten Jahrzehnten mehr und mehr moderne synthetische Rohstoffe, wie z.B. Polyacrylsäure als Gelbildner, zur Herstellung eingesetzt. Dadurch lassen sich die gemeinhin pastösen Cataplasmen auch als Hydrogelmatrices mit verbesserter Anmutung und Anwenderfreundlichkeit darstellen. EP 1 136 057 beschreibt ein wässriges Gelsystem zur kosmetischen Anwendung ohne Träger oder Abdeckung mit einer Lichtdurchlässigkeit von min. 70 %.
In EP 0 507 160 werden Cataplasmen mit Lidocain enthaltend beschrieben.

Nachteilig an den beschriebenen Cataplasmen ist, dass zur Herstellung der Basismatrices viele verschiedene Einzelkomponenten wie Gelbildner, Verdicker, Weichmacher, Feuchthaltemittel, Stabilisatoren, Emulgatoren, pH-Regulatoren, Antioxidantien etc. benötigt werden, bei wirkstoffhaltigen Cataplasmen evtl. noch zusätzlich Lösungsvermittler und Penetrationsbeschleuniger. Da sich Klebverhalten und Konsistenz einer solchen Matrix aus dem Zusammenwirken aller Einzelkomponenten ergeben, gestaltet sich eine gezielte Produktentwicklung/-optimierung hinsichtlich dieser grundlegenden Produktanforderungen entsprechend zeitaufwändig und schwierig.

Die Herstellung von Polymermatrices, insbesondere Gelmatrices, aus Polyacrylaten ist ebenfalls seit vielen Jahren bekannt und wird z. B. in EP 0 507 160, JP 11-228340 und JP 04178323 beschrieben. Gelmatrices werden u.a. als Klebgrundlage und Wirkstoffreservoir in transdermalen Systemen eingesetzt. Solche Systeme haben eine ausreichende Klebkraft, speziell auf feuchter Haut (Bukkalpflaster), lassen sich aufgrund ungenügender Kohäsivität bei Bedarf aber nicht vollständig wieder abziehen.

Polyacrylsäure muss zur Ausbildung eines Gels mit definierter Struktur vernetzt werden. Die Natur des Vernetzers trägt dabei entscheidend zur Struktur des resultierenden Gels bei. Die üblichen vernetzende Agenzien können dabei Metallionen (z.B.: Al³⁺-Ionen), oder organische Verbindungen sein. Die Vernetzung mit Aluminiumsalzen läuft über die Koordination der Sauerstofffunktionen der Polyacrylsäure an die Al³⁺-Ionen. Es bildet sich ein sehr engmaschiges Gel mit hoher Viskosität aus, wobei die Viskosität des Gels nur über die Menge an Vernetzer gesteuert werden kann (handbook of pressure sensitive adhesive technology, Seite 458 ff, 1999).

In JP 11-228340 werden Gele auf Polyacrylsäurebasis offenbart, die als Vernetzer Al⁺³-Verbindungen nutzen. Der Einsatz der zwingend notwendigen Aluminiumverbindung als Vernetzungsagens ist begrenzt, da ansonsten die physikalischen Eigenschaften des Gels verschlechtert werden. Bei zu hohem Anteil an Aluminiumvernetzer wird das Gel zu hart.

Aus der Literatur sind weitere Beispiele der Vernetzung mit multivalenten Metallionen bekannt, z.B. US 3900610 (Zinksalze), US 3770780 oder US 3790533 (Titanverbindungen). Die ionische Vernetzung mit Metallionen führt zu harten, viskosen und wenig klebrigen Polymergelen (handbook of pressure sensitive adhesive technology, Seite 458 ff, 1999).

In EP 303445 wird ein Pflaster mit monolither Gelmatrix auf Basis wasserlöslicher Polymere offenbart. Als zwingend erforderliche Bestandteile sind Cleboprid oder ein pharmazeutisch akzeptables Salz davon als Wirkstoff, Wasser, wasseraufnehmende Agenzien und wasserlösliche Polymere vorgesehen. Als wasserlösliche Polymere kann der Fachmann aus einer Reihe bekannter Polymere wie Polyvinylalkohol, Gelatine, Polyacrylsäure, Natriumpolyacrylate, Methylcellulose, Carboxymethylcellulose, Polyvinylpyrrolidon, Gummi und anderen vernetzbaren Polymeren sowie Mischungen daraus auswählen

EP 976382 beschreibt ein Pflaster enthaltend eine Matrix, bestehend aus einem in wässriger Phase hydrophil gelierendes System, gebildet aus Gelangummi und mindestens einem weiteren Hydrokolloid. Beansprucht wird zwingend Gelangummi. Unter Gelangummi versteht der Fachmann, wie es Fachlexika definieren, Hydrokolloide, die aus folgenden Seepflanzen gewonnen werden: Agardhiella tenera, Furcellaria fastigiata, Hypnea cervicornis, musciformis, spicifera, Suhria vitata. Meeresalgenextrakte sind darunter nicht zu verstehen. Ebenso werden die wesentlichen Aspekte der selbstklebenden Eigenschaften, der Einstellbarkeit von Klebkraft und Elastizität der resultierenden Matrices nicht erwähnt. Ein weiteres Problem bei der Vernetzung von Polyacrylsäure zu einer selbstklebenden Matrix bzw. Gel ist, dass eine einmal hergestellte Matrix mit definierten physikalischen Eigenschaften, Viskosität, Klebrigkeit etc. in einem späteren Herstellungsprozess die gleichen definierten Eigenschaften aufweisen muss. Diese Reproduzierbarkeit ist mit den derzeit bekannten Vernetzungstechnologien aufwändig oder gar nicht zu verwirklichen.

Ein weiteres Problem bei der kosmetischen Hautbehandlung zeigt sich, dass bei unerwünschte Hauterscheinungen diese sich durch die topische Applikation von Wirkstoffen in Form von Emulsionsformulierungen u. ä. nicht optimal behandeln lassen. Insbesondere bei schwieriger zu behandelnden Hauterscheinungen wie Hautfalten, Verlust der Hautelastizität und Altersflecken sowie irritativen Hauterscheinungen wie dem Sonnen- und Rasurbrand etc. fehlt ein kosmetisches Applikationssystem, das eine intensive Darreichung des Wirkstoffs und zugleich wohltuende Effekte auf die Haut zur positiven Beeinflussung des Hautzustandes zeigt.

Aufgabe der vorliegenden Erfindung ist es daher ein einfaches Polymermatrixsystem für Cataplasmen/Hydrogele zu entwickeln, welches mit wenigen Einsatzstoffen gezielt Matrices mit bestimmter Konsistenz und Klebkraft herstellen lässt.
Weitere Aufgabe der vorliegenden Erfindung ist es eine Polymermatrix bereit zu stellen, in die wasserlösliche oder hydrophobe Wirkstoffe eingearbeitet werden können und diese gezielt an die Haut wieder abgegeben werden können.
Des weiteren ist eine Aufgabe der vorliegenden Erfindung Pflaster bzw. kosmetische Matrices zur Verfügung zu stellen, die zuvor genannte Polymermatrices enthalten und als TTS, Pads oder Pflaster verwendet werden können.
Darüber hinaus ist eine Aufgabe der vorliegenden Erfindung eine Gelmatrix bereit zu stellen, die als kosmetische Applikationsform zur Behandlung und auch zur Prophylaxe unerwünschter Hauterscheinungen geeignet ist und den Nachteilen des Standes der Technik abhelfen. Insbesondere soll hierbei der hautpflegende und feuchtigkeitsspendene Aspekt berücksichtigt werden.

Gelöst werden diese Aufgaben durch eine Polymermatrix entsprechend Anspruch 1. In den Unteransprüchen sind bevorzugte Ausführungsformen der Matrices offenbart. Die Erfindung umfasst darüber hinaus auch deren Verwendung.

EP 1172083 offenbart Hydrogelmatrix Partikel und deren Verwendung in auf die Haut einreibbaren Kosmetika.

FR 94977 offenbart eine Polymermischung mit verschiedensten Bestandteilen, wobei als Klebkraft generierende Bestandteile kristalliner Sxchwefel, Vinylklebstoff und Trioxymethylen beschrieben werden.

WO 0102478 offenbart eine selbstklebende Polymermatrix aus Polysacchariden, die zur Gelbildung zwingend mono-oder multivalente Salze enthalten. In der WO 0102478 ist keine die Polymermatrix bildende Polyacrylsäure dargestellt.

DE 10054479 beschreibt selbstklebende Polymermatrices, wobei die Polymere gebildet werden aus einer Wirkstoffdispersion umfassend einen Algenextrakt und einer Dispersion des Klebstoffes (Duro-Tak). Beide Dispersionen umfassen ein organisches Lösemittel sowie die Wirkstoffdispersion umfasst Permeationsverstärker.

US 6190889 offenbart selbstklebende Polymermatrices, die entweder als zwingenden Bestandteil zur Polymerbildung Gelantine enthalten ist und/oder Polyacrylsäure und/oder Meeresalgenextrakte nicht umfassen.

JP 2275470 offenbart keine selbstklebenden Polymermatrices lediglich gebildet aus Polyacylsäure, Meeresalgenextrakt und Alkohol.

WO 9530411 beschreibt Polymermatrices gebildet aus Polyacrylsäure, Agar und Wasser. Die IPN-polymermatrices sind nicht klebend, da eine zusätzliche Klebschicht (adhesive) zur Bildung eines Pflasters oder Pad angeführt wird.

Es war überraschend und für den Fachmann außerordentlich erstaunlich, dass eine selbstklebende Polymermatrix aus einem in Wasser gelbildenden Polymer umfassend mindestens ein Polyacrylsäurepolymer, Wasser, einem mit Alkohol nicht gelbildendem Meeresalgenextrakt und Alkohol, ausgewählt aus Glycerin oder Propandiol, das Bündel an Aufgaben löst.

Überraschenderweise hat sich insbesondere gezeigt, dass
- die beschriebene kosmetische Matrix auch ohne Wirkstoff per se hautpflegende, feuchtigkeitsspendende und kühlende Eigenschaften aufweist und also solche auch schon ohne Wirkstoffeinbringung Anwendung finden kann, um den Nachteilen des Standes der Technik Abhilfe zu verschaffen,
- eine für den Verbraucher sehr praktikable und angenehme Anwendungsform darstellt, da die Oberfläche durch Verwendung eines geeigneten Fliesmaterials im Gegensatz zu vielen kosmetischen und damit getränkten Tüchern trocken und mit einem angenehm seidigen Gefühl erscheint und
- durch Einbringen eines oder mehrerer Wirkstoffe den unerwünschten Hautzustände positiv entgegenwirken oder als Prophylaxe dienen kann.

Die vorliegende Erfindung betrifft eine kosmetische oder dermatologische Formulierung, die insbesondere geeignet ist, kosmetische oder dermatologische Wirkstoffe in einer besonders effektiven Weise der Haut zuzuführen und dies darüber hinaus in einer besonders kosmetischen und für den Anwender angenehmen Form tut.

Die Matrix besteht aus Wasser und einem in Wasser gelbildendem Polymer, bevorzugt Polyacrylsäuregel, als klebkraftbestimmender Komponente. Als Meeresalgenextrakt wird bevorzugt Agar-Agar eingesetzt. Als Alkohol wird bevorzugt Glycerin eingesetzt, das als Konsistenzfaktor wirkt. Obwohl die Einzelkomponenten bekannterweise für die Herstellung von Cataplasmen oder Hydrogelen eingesetzt werden, war es bislang nicht bekannt Agar-Agar in Verbindung mit Glycerin gezielt als Konsistenzfaktoren für Polyacrylsäurematrices einzusetzen.

Durch eine Erhöhung des Anteils an Meeresalgenextrakt in Polymermatrices, wie Cataplasmen/Hydrogelen, wird die Festigkeit der Matrices erhöht. Dies erhöht jedoch auch die Steifigkeit und verringert die Klebrigkeit. Dieser Nachteil kann durch Zusatz von Alkohol, insbesondere von Glycerin, wieder ausgeglichen werden. Es kann somit eine gewünschte Elastizität der resultierenden Polymermatrix bei konstantem Anteil an Meeresalgenextrakt eingestellt werden.

Es zeigte sich demnach eine synergistische Kombination aus Meeresalgenextrakt und Glycerin, um eine gewünschte Elastizität der Gelmatrices zu gewährleisten.
Grundlage für den Einsatz als Konsistenzfaktor ist, dass Meeresalgenextrakt, im Gegensatz zu insbesondere der weit verbreiteten Gelatine und anderen Konsistenzfaktoren, in Verbindung mit Alkoholen, wie Glycerin oder Propandiol, keine Gelbildung hervorruft. Da sich erfindungsgemäße ein- oder mehrwertige Alkohole, Glycerin oder Propandiol, In Wasser homogen verteilen, aber mit dem Meeresalgenextrakt keine Gele bilden, wirken solcher Art Alkohole somit als Elastizitätsfaktor für die Matrices.

Bevorzugt einzusetzender Meeresalgenextrakt ist neben Agar-Agar auch Carrageenan. Carrageenan ist ein hydrophiles Polysaccharid von hohem Molekulargewicht, das aus verschiedenen Rotalgen, vornehmlich Chondrus crispus, durch Heißwasserextraktion, nachfolgendem Ausfrieren und anschließender Reinigung gewonnen wird. Die Struktur von Carrageenan besteht hauptsächlich aus sich wiederholenden Galactose und 3,6 Anhydrogalactoseeinheiten, beide sowohl in sulfatierter wie unsulfatierter Form. Der wichtigste Unterschied zwischen kappa, iota und lambda Carrageenan ist die Anzahl und Position der Estersulfatgruppen an den sich wiederholenden Galactoseeinheiten.
Eine Gelbildung von Carrageenan ist nur in Gegenwart von Kationen möglich. Erfindungsgemäß bevorzugt sind kappa und iota Carrageenan, welche in Gegenwart von Calcium-(kappa und iota), Kalium- und Ammonium-Ionen (nur kappa) Gele bilden. Besonders vorteilhaft ist der Einsatz entsprechender Kationenhydroxide, da die zur Herstellung erfindungsgemäßer Gelmatrixsysteme ebenfalls eingesetzte Polyacrylsäure zur Ausbildung stabiler Gele neutralisiert werden muss.

Industriell angeboten wird Carrageenan z.B. von Lehmann & Voss & Co. unter den Bezeichnungen Gelcarin, Viscarin und Seaspen.

Meeresalgenextrakt, wie erfindungsgemäß besonders bevorzugt Agar-Agar, ist ein hydrophiles Kolloid von Polysaccharid-Struktur bestehend aus dem gelierenden Agarose und dem nichtgelierendern Agaropektin, das aus verschiedenen Meeresalgen der Rhodophyceen-Klasse durch Heißwasserextraktion, nachfolgendem Ausfrieren und anschließender Reinigung gewonnen wird. Industriell angeboten wird Agar-Agar z.B. von der Riedel de Haen AG.

Der Extrakt, insbesondere Agar-Agar oder Carrageenan, wird bevorzugt in einer Menge von 0,1 - 15 Gew.%, besonders bevorzugt zwischen 0,5 - 5 Gew.%, eingesetzt. Alle Prozentangaben beziehen sich dabei auf Gewichtsanteile der Polymermatrix sofern nicht Gegenteiliges angegeben ist.

Ein- oder mehrwertige Alkohole wie z.B. Glycerin (1,2,3-Propantriol), sind unter anderem als Lösungsvermittler oder Feuchthaltemittel weit verbreitet eingesetzte Hilfsstoffe der pharmazeutischen Industrie.
Ein- oder mehrwertigen Alkohole, wie z.B. Glycerin, werden erfindungsgemäß bevorzugt in einer Menge von 1 - 85 Gew.%, besonders bevorzugt zwischen 5 - 45 Gew.% eingesetzt.
Der Anteil an in Wasser gelbildendem Polymer wie z.B. Polyacrylsäuregel in der Matrix regelt das Haftvermögen. Im Gegensatz zu Agar-Agar bildet Polyacrylsäure aber sowohl mit Wasser wie auch mit Alkoholen Gele, so dass das durch den Anteil an Polyacrylsäure eingestellte Haftvermögen unabhängig vom jeweiligen Alkoholanteil konstant bleibt.

Erfindungsgemäß vorteilhafte Polyacrylate sind Acrylat-Alkylacrylat-Copolymere, insbesondere solche, die aus der Gruppe der sogenannten Carbomere oder Carbopole (Carbopol® ist eine eingetragene Marke der B. F. Goodrich Company) gewählt werden. Insbesondere zeichnen sich das oder die erfindungsgemäß vorteilhaften Acrylat-Alkylacrylat-Copolymere durch die folgende Struktur aus:

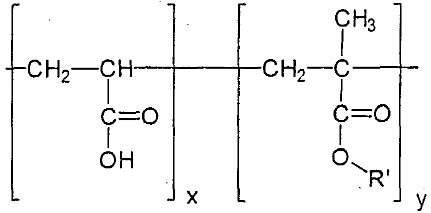

Darin stellen R' einen Alkylrest, insbesondere einen langkettigen Rest, und x und y Zahlen dar, welche den jeweiligen stöchiometrischen Anteil der jeweiligen Comonomere symbolisieren.

Erfindungsgemäß besonders bevorzugt sind Acrylat-Copolymere und/oder Acrylat-Alkylacrylat-Copolymere, welche unter den Handelbezeichnungen Carbopol® 1382, Carbopol® 981 und Carbopol® 5984 von der B. F.Goodrich Company erhältlich sind, bevorzugt Polyacrylate aus der Gruppe der Carbopole der Typen 980, 981, 1382, 2984, 5984 sowie besonders bevorzugt Carbomer 2001.
Ferner vorteilhaft sind Copolymere aus C₁₀₋₃₀-Alkylacrylaten und einem oder mehreren Monomeren der Acrylsäure, der Methacrylsäure oder deren Ester, die kreuzvernetzt sind mit einem Allylether der Saccharose oder einem Allylether des Pentaerythrit.

Das in Wasser gelbildende Polymer, insbesondere Polyacrylsäure und/oder deren Copolymere, werden bevorzugt in einer Menge von 2 - 55 Gew.%, besonders bevorzugt zwischen 5 - 30 Gew.% eingesetzt.

Die Herstellung der Polymermatrices erfolgt ohne Verwendung organischer Lösemittel, vorzugsweise bei 40 - 95°C, in handelsüblichen Mischern/Knetem oder kontinuierlich in geeigneten Extrudern.
Als in Wasser gelbildendes Polymer eignet sich u.a. auch Affenbrotbaummehl.

Auf diese Weise lassen sich nur unter Verwendung von Wasser, in Wasser gelbildendem Polymer, wobei das in Wasser gelbildende Polymer aus mindestens einem Polyacrylsäurepolymer besteht, Meeresalgenextrakt und Glycerin und/oder Propandiol als Ausgangsmaterialien gezielt weiche, geschmeidige, selbstklebende Hydrogelmatrices als Basis zur Herstellung und Anwendung als Pflaster, TTS, Cataplasmen oder kosmetischen Pads / Matrices herstellen.

Zur Ausfertigung besonderer anwendungstechnischer Eigenschaften können die Polymermatrices mit entsprechenden Weichmachern, Lösungsvermittlern, Penetrationsenhancern, Neutralisationsmitteln wie z.B. Tromethamol (2-Amino-2-(hydroxymethyl)-1,3-propandiol), Triethanolamin (2,2',2"-Nitrilotriethanol) oder NaOH, Füllstoffen und/oder anderen bekannten Zusätzen versetzt werden, deren Zusatz jedoch nicht zwingend ist.

In einer erfindungsgemäß besonders bevorzugten Ausführungsform enthält die Polymermatrix bzw. Gelmatrix dermatologische oder kosmetische Wirkstoffe zur kontrollierten lokalen bzw. systemischen Abgabe an/in die Haut, in Mengen von 0 - 35 Gew.%, bevorzugt 0 - 15 Gew.%.

Die Gelmatrix kann somit mit hydrophilen, bei geeignetem Lösungsvermittler auch hydrophoben, Wirkstoffen zur kosmetischen Behandlung von unerwünschten Hauterscheinungen dotiert werden. Bei der Einarbeitung hydrophober Wirkstoffe kann es von Nutzen sein, Cyclodextrine zur Verkapselung einzusetzen.

Cyclodextrine (Cycloamylosen, Cycloglucane) sind in kosmetischen und pharmazeutischen Zubereitungen an sich bekannt.
Die Verbesserung der Löslichkeit schwerlösliche Substanzen in Gegenwart von Cyclodextrinen in wässrigem Milieu ist für einzelne Substanzen beschrieben. Vorteilhaft können sowohl die Einschlußverbindungen einer Substanz, auch Gast genannt, mit einer Cyclodextrinspezies, wobei sowohl 1:1 oder 1:2 Komplexe, wie auch Komplexe mit weiteren molaren Verhältnissen (Gast: Cyclodextrin) möglich sind, sowie auch deren physikalische Mischung sein.
Es handelt sich bei den Cyclodextrinen um zyklische Oligosaccharide bestehend aus α-1,4 verknüpften Glucosebausteinen. In der Regel sind sechs bis acht Glucosebausteine (α-, β-, bzw. γ-Cyclodextrin) miteinander verbunden.
Cyclodextrine werden bei Einwirkung von *Bacillus macerans* auf Stärke erhalten. Sie besitzen einen hydrophoben Innenraum und eine hydrophile Außenseite. Cyclodextrine und ihre Derivate können aufgrund Ihrer Struktur Inklusionskomplexe bilden. Sie sind zur "molekularen Verkapselung" von Wirkstoffen geeignet, z.B. als schützende Umhüllung empfindlicher Moleküle in kosmetischen und pharmazeutischen Formulierungen.
Diese Anwendungen sind auch in einer Reihe von Patenten beschrieben, z.B.:.WO 98/55148, EP 0 579 435, EP 0 392 608. In diesen Schriften wird jedoch meist nur ein Wirkstoff vom Cyclodextrin (-derivat) komplexiert. Mehrfachkomponenten-Inklusionskomplexe werden zwar in EP 0756 493 beschrieben, doch handelt es sich hier bei näherer Betrachtung um ein Salz und nicht um eine Zweikomponentenmischung von Säure und Base.

Mit "Cyclodextrin und/oder ein Derivat davon" sind im folgenden sowohl Cyclodextrine mit unterschiedlicher Anzahl von Glucosebausteinen im Ringmolekül als auch Derivate dieser Verbindungen gemeint.

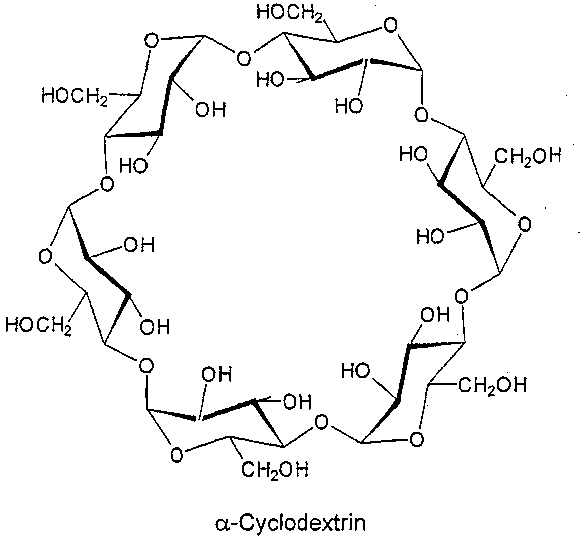

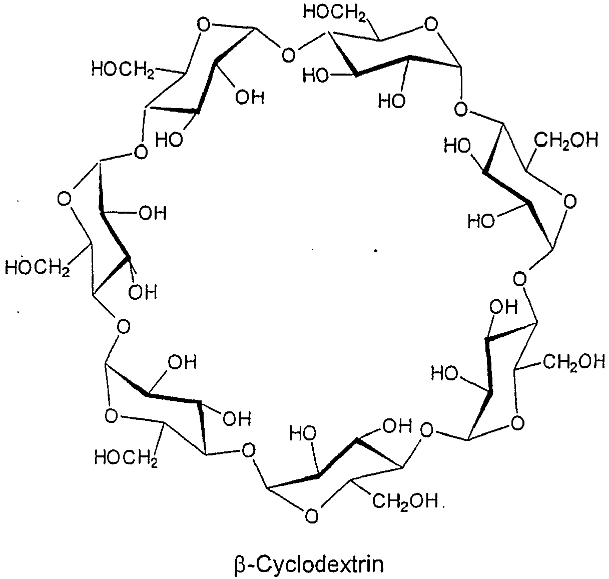

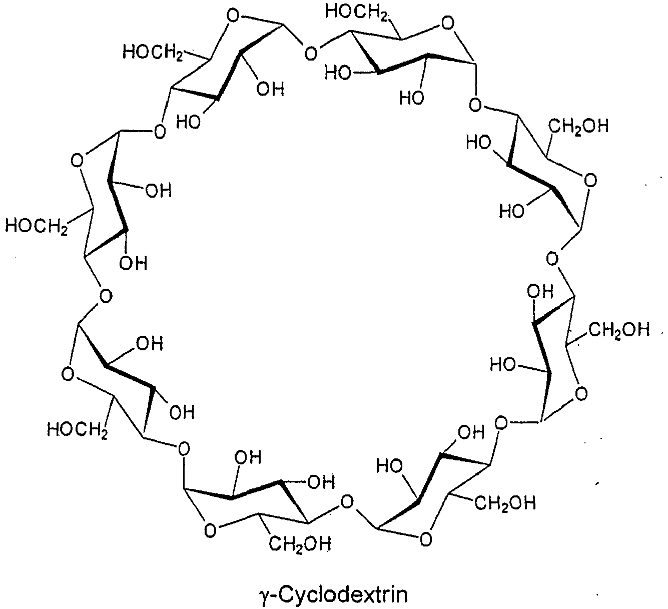

Erfindungsgemäß werden das oder die Cyclodextrine bevorzugt in kosmetischen oder dermatologischen Zusammensetzungen eingesetzt in einer Konzentration von 0.0005 bis 20.0 Gewichts-%, insbesondere 0,01 bis 10 Gew.- % und besonders bevorzugt in einer Konzentration von 0.1 bis 5.0 Gew.-%.

Es ist erfindungsgemäß vorteilhaft native, polar- und/oder unpolar- substituierte Cyclodextrine einzusetzen. Hierzu gehören vorzugsweise aber nicht ausschließlich Methyl-, insbesondere random-Methyl-β-Cyclodextrin, Ethyl- sowie Hydroxypropyl-Cyclodextrine, beispielsweise HP-□-Cyclodextrin oder HP-□-Cyclodextrin.

Die erfindungsgemäß besonders bevorzugten Cyclodextrinspezies sind γ-Cyclodextrin sowie Hydroxypropyl-β-Cyclodextrin.

Ein weiterer Stand der Technik ist in folgenden Schriften enthalten:
K. Uekama et al., Chemical Reviews, 1998, 98, 2045-2076, "Cyclodextrin drug carrier systems"
T. Loftsson, Int. J. Dermatology, 1998, 37, 241-246, "Cyclodextrins: new drugdelivery systems in dermatology".
J. Zatz et al. Cosmetics & Toiletries, 1997, 112, Juli, S. 39ff, "Applications of cyclodextrins in skin products.
U. Citernesi, Cosmetics & Toiletries, 1995, 110, März, S. 53 ff, Cyclodextrins in functional dermocosmetics.

Die erfindungsgemäß verwendeten Cyclodextrine bzw. Cyclodextrin-Gast-Inklusionskomplexe bzw. die Cyclodextrin-Substanz Mischungen lassen sich ohne Schwierigkeiten in die Polymermatrix einarbeiten.

Da es sich bei der erfindungsgemäßen Matrix um eine wasserhaltige Applikationsform handelt, erreicht man zusätzlich einen kühlenden Effekt, der per se schon kosmetisch angenehm ist und zum Wohlbefinden beiträgt. Diese positive Wirkung kann durch die Zugabe weiterer pflegender Bestandteile verstärkt werden. Neben Glycerin können insbesondere Serinol (3-Amino-1,2-Propandiol) bzw. Isoserinol (2-Amino-1,3-Propandiol) sowie Harnstoff und PCA (Pyrrolidoncarbonsäure) als feuchtigkeitsspendende Substanzen beigefügt werden. Selbstverständlich können auch weitere Substanzen zu diesem Zwecke beigefügt werden.

Als besonders geeignete Wirkstoffe im Sinne der Erfindung können den genannten kosmetischen Matrices / Pads folgende Wirkstoffe entweder einzeln oder auch in Kombination beigefügt werden.

Bei der vorliegenden Erfindung hat sich völlig überraschenderweise gezeigt, dass die erfindungsgemäßen Formulierungen sich auch ganz besonders eignen für den Einsatz von Wirkstoffen, die den Zustand der Haut positiv beeinflussen. So zeigte sich, dass Wirkstoffe zur positiven Beeinflussung der Altershaut, die die Entstehung von Falten oder auch bestehenden Falten vermindern. Als besonders bevorzugte Wirkstoffe gelten daher Biochinone, insbesondere Ubichinon Q10, Kreatin, Kreatinin, Carnitin, Acetylcarnitin, Biotin, Isoflavon und Isoflavonoide, Genistein, Arctiin, Cardiolipin, Liponsäure, Anti Freezing Proteine, Hopfen- und Hopfen-Malz-Extrakte, und/oder die Restrukturierung des Bindegewebes fördernde Stoffe, Isoflavonoide sowie Isoflavonoid-haltige Pflanzenextrakte wie z.B. Soja- und Klee-Extrakte, die in den erfindungsgemäßen Matrices sehr gut verwendet werden können. Auch zeigte sich, dass sich die Matrix in besonderer Weise eignet, Wirkstoffe zur Unterstützung der Hautfunktionen bei trockener Haut, wie beispielsweise Vitamin C, Biotin, Carnitin, Kreatin, Kreatinin, Propionsäure, Glycerin, Grüntee-Extrakte, Eucalyptusöl, Harnstoff und Mineralsalze wie z. B. NaCl, Meeresmineralien sowie Osmolyte wie z. B. Taurin, Inositol, Betain, quartäre Ammoniumverbindungen, zu verwenden. In ähnlicher Weise erwies sich die Einarbeitung von Wirkstoffen zur Linderung bzw. positiven Beeinflussung von irritativen Hautzuständen, sei es bei empfindlicher Haut im allgemeinen oder bei durch Noxen gereizter Haut (UV-Licht, Chemikalien), als vorteilhaft. Hier sind Wirkstoffe zu nennen wie Sericoside, verschiedene Extrakte des Süssholzes, Licochalcone A, Silymarin bzw. Silyphos, Dexpanthenol, Ethanol, Inhibitoren des Prostaglandinstoffwechsels, insbesondere der Cyclooxygenase, und des Leukotrienstoffwechsels, insbesondere der 5-Lipoxygenase, aber auch des 5-Lipoxygenase Inhibitor Proteins, FLAP. Auch erwies sich die Einarbeitung von Modulatoren der Pigmentierung als vorteilhaft. Hier sind Wirkstoffe zu nennen, die die Pigmentierung der Haut vermindern und so zu einer kosmetisch gewünschten Aufhellung der Haut führen und/oder das Auftreten von Altersflecken reduzieren und/oder bestehende Altersflecken aufhellen, wie Tyrosinsulfat, Dioic acid (8-Hexadecen-1,16-dicarbonsäure), Liponsäure und Liponamid, verschiedene Extrakte des Süssholzes, Kojisäure, Hydrochinon, Arbutin, Fruchtsäuren, insbesondere Alpha-Hydroxy-Säuren (AHAs), Bearberry (Uvae ursi), Ursolsäure, Ascorbinsäure, Grüntee-Extrakte, Aminoguanidin und/oder Pyridoxamin. In gleicher Weise erwiesen sich die erfindungsgemäßen Matrices als hervorragende Grundlage für Wirkstoffe, die eine verstärkte/schnellere Bräunung der Haut herbeiführen (Advanced Glycation Endproducts (AGE), Lipofuscine, Nukleinsäure-Oligonukleotide, Purine und Pyrimidine, NOfreisetzende Substanzen, sei es mit oder ohne Einfluss von UV-Licht.

Die Polymermatrix wird den oder die Wirkstoffe in Mengen von 0 - 35 Gew.%, bevorzugt 0 - 15 Gew.%, ganz besonders bevorzugt 0,02-2% enthalten.

Zur Prophylaxe vor oxidativen und degenerativen Schäden und insbesondere zur Behandlung von denselben hat es sich als überraschenderweise als sinnvoll erwiesen, den kosmetischen Matrices/Pads Antioxidantien hinzuzufügen. Vorteilhaft werden die Antioxidantien gewählt aus der Gruppe bestehend aus Aminosäuren, z.B. Glycin, Lysin, Arginin, Cystein, Histidin, Tyrosin, Tryptophan, und deren Derivate (als Salz-, Ester-, Ether-, Zucker-, Nukleotid-, Nukleosid-; Peptid- und Lipid-Verbidung), Imidazole, z.B. Urocaninsäure, und deren Derivate, als Salz-, Ester-, Ether-, Zucker-, Nukleotid-, Nukleosid-, Peptid- und/oder Lipid-Verbidung, Peptide wie D,L-Carnosin, D-Carnosin, L-Carnosin, Anserin und deren Derivate, z.B. als Salz-, Ester-, Ether-, Zucker-, Thiol-, Nukleotid-, Nukleosid-, Peptid- und Lipid-Verbidung, Carotinoide, Carotine, z.B. α-Carotin, β -Carotin, ψ-Lycopin, Phytoen, und deren Derivate, z. B. als Salz-, Ester-, Ether-, Zucker-, Nukleotid-, Nukleosid-, Peptid- und/oder Lipid-Verbidung, Chlorogensäure und deren Derivate, als Salz-, Ester-, Ether-, Zucker-, Thiol-, Nukleotid-, Nukleosid-, Peptid-und/oder Lipid-Verbidung, Aurothioglucose, Propylthiouracil und andere Thiole, z.B. Thioredoxin, Liponsäure, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester, sowie deren Salze, Dilaurylthiodipropionat, Distearylthiodipropionat, Thiodipropionsäure und deren Derivate, als Salz-, Ester-, Ether-, Zucker-, Thiol-, Nukleotid-, Nukleosid-, Peptid- und/oder Lipid-Verbidung, sowie Sulfoximinverbindungen, z.B. Homocysteinsulfoximin, Buthioninsulfone, Penta-, Hexa-, Heptathioninsulfoximin, in sehr geringen verträglichen Dosierungen, z.B. pmol bis µmol/kg. Ferner (Metall)-Chelatoren, z.B. Apoferritin, Desferral, Lactoferrin, α-Hydroxyfettsäuren, Palmitinsäure, Phytinsäure, und deren Derivate, als Salz-, Ester-, Ether-, Zucker-, Thiol-, Nukleotid-, Nukleosid-, Peptid- und/oder Lipid-Verbidung, α-Hydroxysäuren, z.B. Citronensäure, Milchsäure, Apfelsäure, Huminsäure, Gallensäure, Gallenextrakte, Bilirubin, Biliverdin, Melanin, EDTA, EGTA und deren Derivate, ungesättigte Fettsäuren und deren Derivate, z.B. γ-Linolensäure, Linolsäure, Ölsäure, Folsäure und deren Derivate, Furfurylidensorbitol und dessen Derivate, Ubichinon, Ubichinol, Plastochinon und deren Derivate, als Salz-, Ester-, Ether-, Zucker-, Thiol-, Nukleotid-, Nukleosid-, Peptid- und Lipid-Verbidung, Vitamin C und Derivate, z.B. Ascorbylpalmitat, Mg-Ascorbylphosphat, Ascorbylacetat, Tocopherole und Derivate, z.B. Vitamin-E-acetat, Trolox^{®}, sowie Phenolische Verbindungen und Pflanzenextrakte, diese enthaltend, wie z. B. Flavonoide, z: B. Glycosylrutin, Ferulasäure, Kaffeesäure, Furfurylidenglucitol, Butylhydroxytoluol, Butylhydroxyanisol, Nordihydroguajakharzsäure, Nordihydroguajaretsäure, Trihydroxybutyrophenon und deren Derivate, als Salz-, Ester-, Ether-, Zucker-, Nukleotid-, Nukleosid-, Peptid- und Lipid-Verbidung. Harnsäure und deren Derivate, Mannose und deren Derivate, als Salz-, Ester-, Ether-, Zucker-, Thiol-, Nukleotid-, Nukleosid-, Peptid- und Lipid-Verbidung. Zink und dessen Derivate, z.B. ZnO, ZnSO₄, Selen und dessen Derivate, z.B. Selenmethionin, Ebselen, Stilbene und deren Derivate, z.B. Stilbenoxid, Trans-Stilbenoxid, und die erfindungsgemäß geeigneten Derivate, als Salz-, Ester-, Ether-, Zucker-, Thiol-, Nukleotid-, Nukleosid-, Peptid- und/oder Lipid-Verbidung, dieser genannten Wirkstoffe.

Die Polymermatrix wird den oder die Antioxidantien in Mengen von 0 - 35 Gew.%, bevorzugt 0 - 15 Gew.%, ganz besonders bevorzugt 0,02-2% enthalten.

Als Wirkstoffe können desweiteren beispielsweise ätherische Öle eingesetzt werden. Unter ätherischen Ölen sind aus Pflanzen gewonnene Konzentrate zu verstehen, die als natürliche Rohstoffe hauptsächlich in der Parfüm- und Lebensmittelindustrie eingesetzt werden und die mehr oder weniger aus flüchtigen Verbindungen bestehen. Als Beispiele für diese Verbindungen können 1,8-Cineol, Limonen, Menthol, Borneol und Kampfer genannt werden. Oft wird der Begriff ätherische Öle für die noch in den Pflanzen enthaltenen flüchtigen Inhaltsstoffe verwendet. Im eigentlichen Sinn versteht man aber unter ätherischen Ölen Gemische aus flüchtigen Komponenten, die durch Wasserdampfdestillation aus pflanzlichen Rohstoffen hergestellt werden.

Ätherische Öle bestehen ausschließlich aus flüchtigen Komponenten, deren Siedepunkte in der Regel zwischen 150 und 300 °C liegen. Sie enthalten überwiegend Kohlenwasserstoffe oder monofunktionelle Verbindungen wie Aldehyde, Alkohole, Ester, Ether und Ketone. Stammverbindungeri sind Mono- und Sesquiterpene, Phenylpropan-Derivate und längerkettige aliphatische Verbindungen.

Bei manchen ätherischen Öle dominiert ein Inhaltsstoff, zum Beispiel Eugenol in Nelkenöl mit mehr als 85%, andere ätherische Öle stellen hingegen komplex zusammengesetzte Mischungen der einzelnen Bestandteile dar. Oft werden die organoleptische Eigenschaften nicht von den Hauptkomponenten, sondern von Nebenoder Spurenbestandteilen geprägt, wie zum Beispiel von den 1,3,5-Undecatrienen und Pyrazinen im Galbanum-Öl. Bei vielen der kommerziell bedeutenden ätherischen Öle geht die Zahl der identifizierten Komponenten in die Hunderte. Sehr viele Inhaltsstoffe sind chiral, wobei sehr oft ein Enantiomer überwiegt oder ausschließlich vorhanden ist, wie zum Beispiel (-)-Menthol im Pfefferminzöl oder (-)-Linalylacetat im Lavendelöl.

Als bevorzugte ätherische Öle können Oleum Eucalypti, Oleum Menthae piperitae, Oleum camphoratum, Oleum Rosmarini, Oleum Thymi, Oleum Pini sibricum und Oleum Pini silverstris sowie die Terpene 1,8-Cineol und Levomethanol genannt werden.

Als weitere ätherische Öle sind Oleum Abietis albae, Oleum Anisi, Oleum Aurantii Floris, Oleum Bergamottae, Oleum Calendulae infusum, Oleum camphoratum, Oleum Caryophylli; Oleum Chamomillae, Oleum Cinnamomi ceylanici, Oleum Citri, Oleum Citronellae, Oleum Cupressi, Oleum Cymbopogonis, Oleum Jecoris, Oleum Lavendulae, Oleum Macidis, Oleum Majoranae, Oleum Melaleucae viridiflorae, Oleum Melissae, Oleum Menthae arvensis, Oleum Menthae piperatae, Oleum Millefolium, Oleum Myrrhae, Oleum Myrte, Oleum Oregani, Oleum Pini sibricum, Oleum Pinisilvestris, Oleum Salviae, Oleum Santali, Oleum Terebinthinae rectificat., Oleum Thymi Oleum Valerianae, Oleum Zingiberis und/oder Teebaumöl zu nennen.

Pfefferminzöle sind durch Wasserdampfdestillation aus Blättern und Blütenständen verschiedener Pfefferminze-Sorten gewonnene ätherische Öle, gelegentlich auch solche aus Mentha arvensis.

Citrusöle sind ätherische Öle, die aus den Schalen von Citrusfrüchten (Bergamotte, Grapefruit, Limette, Mandarine, Orange, Zitrone) gewonnen werden, oft auch Agrumenöle genannt.

Citrusöle bestehen zu einem großen Teil aus Monoterpen-Kohlenwasserstoffen, hauptsächlich Limonen (Ausnahme: Bergamottöl, das nur ca. 40% enthält).

Beispielsweise kann Menthol zur Oberflächenanästhesierung bei Hautirritationen durch leichte Verbrennungen eingesetzt werden. Die so hergestellten Produkte erzeugen ein angenehmes Kältegefühl und können zur Kühlung von Hautreizungen, z.B. leichter Sonnenbrand und Rasurbrand, die keiner fachärztlichen Behandlung bedürfen, zum Einsatz kommen.

Menthol hat drei asymmetrische C-Atome und kommt demzufolge in vier diastereomeren Enantiomerenpaaren vor (vgl. die Formelbilder, die anderen vier Enantiomeren sind die entsprechenden Spiegelbilder).

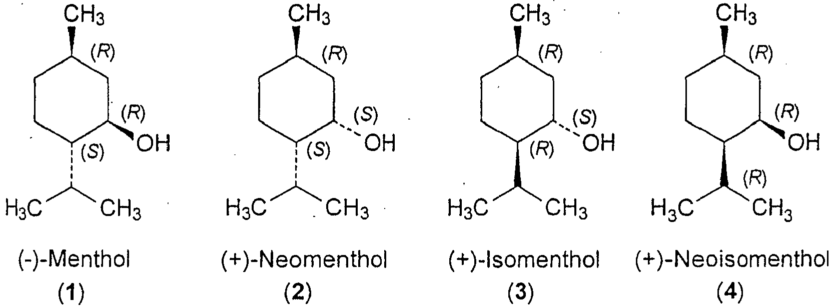

Die Diastereomeren, die destillativ getrennt werden können, werden als Neoisomenthol, Isomenthol, Neomenthol [(+)-Form: Bestandteil des japanischen Pfefferminzöls] und Menthol bezeichnet. Wichtigstes Isomer ist (-)-Menthol (Levomenthol), glänzende, stark pfefferminzartig riechende Prismen.

Als weitere Wirkstoffe kann zum Beispiel Campher zur Behandlung von Hautirritationen/ leichten Schmerzen, Neuralgien und Entzündungen der Matrix zugesetzt werden. Unter Campher versteht man 2-Bornanon, 1,7,7-Trimethylbicyclo[2.2.1]heptan-2-on, siehe untere Abbildung.

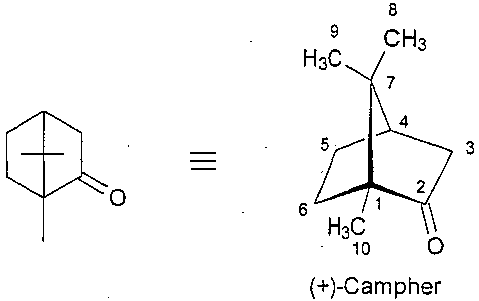

Daneben können für vorteilhafte Ausführungsformen erfindungsgemäßer Hydrogele/Cataplasmen auch hyperämisierende Wirkstoffe wie natürliche Wirkstoffe des Cayenne-Pfeffers oder synthetische Wirkstoffe wie Nonivamid, Nicotinsäurederivate, bevorzugt Bencylnicotinat oder Propylnicotinat, genannt werden beziehungsweise Antiphlogistika und/oder Analgetika.

### Beispielhaft seien Capsaicin

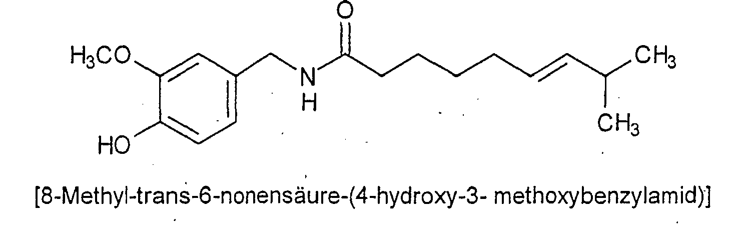

### Nonivamid

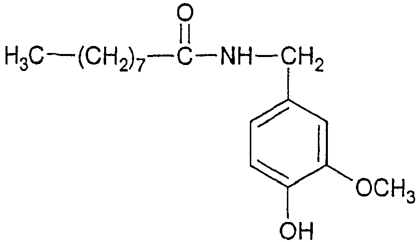

### Nicotinsäurebenzylester

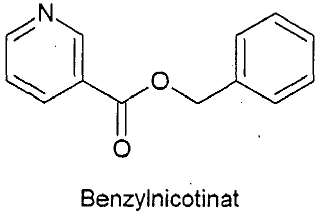

genannt.

Auch Flavon und seine Derivate, oft auch kollektiv "Flavone" genannt, sind vorteilhafte Zusatzstoffe im Sinne der vorliegenden Erfindung. Sie sind durch folgende Grundstruktur gekennzeichnet (Substitutionspositionen angegeben):

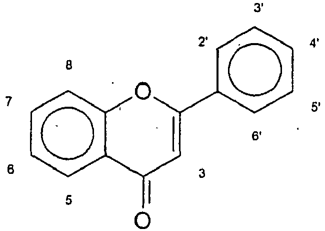

Einige der wichtigeren Flavone, welche auch bevorzugt in erfindungsgemäßen Zubereitungen eingesetzt werden können, sind in der nachstehenden Tabelle aufgeführt:

| | OH-Substitutionspositionen | | | | | | | |
|---|---|---|---|---|---|---|---|---|
| | 3 | 5 | 7 | 8 | 2' | 3' | 4' | 5' |
| Flavon | - | - | - | - | - | - | - | - |
| Flavonol | + | - | - | - | - | - | - | - |
| Chrysin | - | + | + | - | - | - | - | - |
| Galangin | + | + | + | - | - | - | - | - |
| Apigenin | - | + | + | - | - | - | + | - |
| Fisetin | + | - | + | - | - | + | + | - |
| Luteolin | - | + | + | - | - | + | + | - |
| Kampferol | + | + | + | - | - | - | + | - |
| Quercetin | + | + | + | - | - | + | + | - |
| Morin | + | + | + | - | + | - | + | - |
| Robinetin | + | - | + | - | - | + | + | + |
| Gossypetin | + | + | + | + | - | + | + | - |
| Myricetin | + | + | + | - | - | + | + | + |

In der Natur kommen Flavone in der Regel in-glycosidierter Form vor.

Erfindungsgemäß werden die Flavonoide bevorzugt gewählt aus der Gruppe der Substanzen der generischen Strukturformel 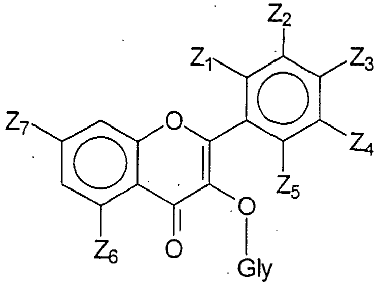 wobei Z₁ bis Z₇ unabhängig voneinander gewählt werden aus der Gruppe H, OH, Alkoxy-sowie Hydroxyalkoxy-, wobei die Alkoxy- bzw. Hydroxyalkoxygruppen verzweigt und unverzweigt sein und 1 bis 18 C-Atome aufweisen können, und wobei Gly gewählt wird aus der Gruppe der Mono- und Oligoglycosidreste.

Erfindungsgemäß können die Flavonoide aber auch vorteilhaft gewählt werden aus der Gruppe der Substanzen der generischen Strukturformel 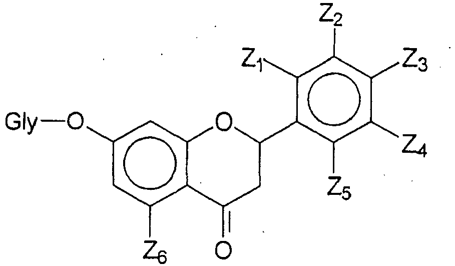 wobei Z₁ bis Z₆ unabhängig voneinander gewählt werden aus der Gruppe H, OH, Alkoxysowie Hydroxyalkoxy-, wobei die Alkoxy- bzw. Hydroxyalkoxygruppen verzweigt und unverzweigt sein und 1 bis 18 C-Atome aufweisen können, und wobei Gly gewählt wird aus der Gruppe der Mono- und Oligoglycosidreste.

Bevorzugt können solche Strukturen gewählt werden aus der Gruppe der Substanzen der generischen Strukturformel 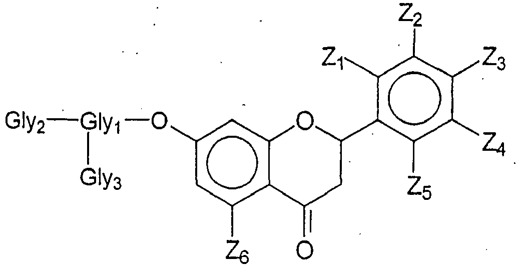 wobei Gly₁, Gly₂ und Gly₃ unabhängig voneinander Monoglycosidreste oder darstellen. Gly₂ bzw. Gly₃ können auch einzeln oder gemeinsam Absättigungen durch Wasserstoffatome darstellen.

Bevorzugt werden Gly₁, Gly₂ und Gly₃ unabhängig voneinander gewählt aus der Gruppe der Hexosylreste, insbesondere der Rhamnosylreste und Glucosylreste. Aber auch andere Hexosylreste, beispielsweise Allosyl, Altrosyl, Galactosyl, Gulosyl, Idosyl, Mannosyl und Talosyl sind gegebenenfalls vorteilhaft zu verwenden. Es kann auch erfindungsgemäß vorteilhaft sein, Pentosylreste zu verwenden.

Vorteilhaft werden Z₁ bis Z₅ unabhängig voneinander gewählt aus der Gruppe H, OH, Methoxy-, Ethoxy- sowie 2-Hydroxyethoxy-, und die Flavonglycoside haben die Struktur

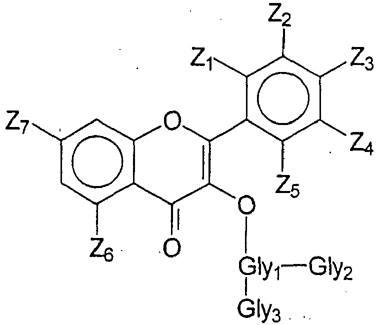

Besonders vorteilhaft werden die erfindungsgemäßen Flavonglycoside aus der Gruppe, welche durch die folgende Struktur wiedergegeben werden: 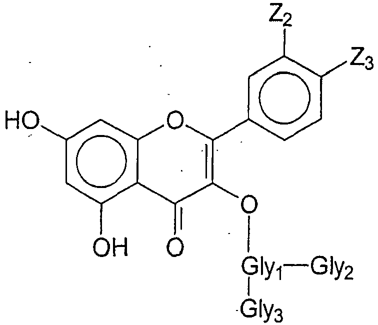 wobei Gly₁, Gly₂ und Gly₃ unabhängig voneinander Monoglycosidreste oder Oligoglycosidreste darstellen. Gly₂ bzw. Gly₃ können auch einzeln oder gemeinsam Absättigungen durch Wasserstoffatome darstellen.

Bevorzugt werden Gly₁, Gly₂ und Gly₃ unabhängig voneinander gewählt aus der Gruppe der Hexosylreste, insbesondere der Rhamnosylreste und Glucosylreste. Aber auch andere Hexosylreste, beispielsweise Allosyl, Altrosyl, Galactosyl, Gulosyl, Idosyl, Mannosyl und Talosyl sind gegebenenfalls vorteilhaft zu verwenden. Es kann auch erfindungsgemäß vorteilhaft sein, Pentosylreste zu verwenden.

Besonders vorteilhaft im Sinne der vorliegenden Erfindung ist, das oder die Flavonglycoside zu wählen aus der Gruppe α-Glucosylrutin, α-Glucosylmyricetin, α-Glucosylisoquercitrin, α-Glucosylisoquercetin und α-Glucosylquercitrin.

Erfindungsgemäß besonders bevorzugt ist α-Glucosylrutin.

Erfindungsgemäß vorteilhaft sind auch Naringin (Aurantiin, Naringenin-7-rhamnogluco-sid), Hesperidin (3',5,7-Trihydroxy-4'-methoxyflavanon-7-rutinosid, Hesperidosid, Hespe-retin-7-O-rutinosid). Rutin (3,3',4',5,7-Pentahydroxyflyvon-3-rutinosid, Quercetin-3-rutino-sid, Sophorin, Birutan, Rutabion, Taurutin, Phytomelin, Melin), Troxerutin (3,5-Dihydroxy-3',4',7-tris(2-hydroxyethoxy)-flavon-3-(6-O-(6-deoxy-α-L-mannopyranosyl)-β-D-gluco-pyranosid)), Monoxerutin (3,3',4',5-Tetrahydroxy-7-(2-hydroxyethoxy)-flavon-3-(6-O-(6-deoxy-α-L-mannopyranosyl)-β-D-glucopyranosid)), Dihydrorobinetin (3,3',4',5',7-Penta-hydroxyflavanon), Taxifolin (3,3',4',5,7-Pentahydroxyflavanon), Eriodictyol-7-glucosid (3',4',5,7-Tetrahydroxyflavanon-7-glucosid), Flavanomareïn (3',4',7,8-Tetrahydroxyflavanon-7-glucosid) und Isoquercetin (3,3',4',5,7-Pentahydroxyflavanon-3-(β-D-Glucopyranosid) oder deren Derivate.

Weitere bevorzugte Wirkstoffklassen einer erfindungsgemäßen Gelmatrix sind - ohne den Anspruch der Vollständigkeit im Rahmen der vorliegenden Erfindung zu erheben:
Antimykotika, wie z.B. Nafitin, Amorrolfin, Tolnaftat, Ciclopirox
Nichtsteroidale Antirheumatika, wie z.B. Glykolsalicylat, Flufenaminsäure, Ibuprofen, Etofenamat, Ketoprofen, Piroxicam, Indomethacin
Antiphlogistika, wie Acetylsalicylsäure
Antipuriginosa, wie z.B. Polidocanol, Isoprenalin, Crotamiton
Lokalanästhetika, wie z.B. Lidocain, Benzocain
Antipsoriatika, wie z.B. Ammoniumbitumasulfonat
Keratolytika, wie z.B. Harnstoff

Von besonderer Bedeutung unter den Wirkstoffen sind für erfindungsgemäße Polymermatrices, Hydrogele/Cataplasmen oder kosmetischen Pads die Desinfektionsmittel beziehungsweise Antiseptika hervorzuheben.
Als Desinfektionsmittel werden Stoffe bezeichnet, die zur Desinfektion, d. h., zur Bekämpfung pathogener Mikroorganismen, zum Beispiel Bakterien, Viren, Sporen, Kleinund Schimmelpilze, geeignet sind. Im allgemeinen werden die Mittel an der Oberfläche von Haut, Kleidung, Geräten, Räumen, aber auch von Trinkwasser, Nahrungsmitteln, Saatgut (Beizen) und als Bodendesinfektionsmittel angewendet.

Besonders lokal anzuwendende Desinfektionsmittel, zum Beispiel zur Wunddesinfektion, werden auch als Antiseptika bezeichnet.

Desinfektionsmittel werden definiert als Stoffe oder Stoffgemische, die bei der Anwendung auf Gegenständen oder Oberflächen diese in einen Zustand versetzen, dass sie keine Infektion mehr verursachen. Ihre Wirkung muss bakterizid, fungizid, viruzid und sporizid, d.h. der Sammelbegriff: mikrobizid, sein. Ein Effekt im Sinne der Bakteriostase ist für Desinfektionsmittel unzureichend. Sie sind daher im allgemeinen pantoxisch, d. h. sie entfalten ihre Wirkung gegen alle lebenden Zellen.

Je nach Verwendungszweck teilt man die Desinfektionsmittel ein in solche zur Wäsche-, Flächen-, Instrumenten-, Haut- und Hände- sowie zur Stuhl- und Sputumdesinfektion. Unter Desinfektionsreiniger versteht man solche Desinfektionsmittel, die auch als Reinigungs- und gegebenenfalls Pflegemittel fungieren.

Unter Berücksichtigung der vielfältigen Forderungen, die an Desinfektionsmittel gestellt werden, wie zum Beispiel breites Wirkungsspektrum, kurze Einwirkungszeiten, Hautverträglichkeit, geringe Toxizität, Materialverträglichkeit usw. kommen nur einige Wirkstoff-Typen für den gewünschten Einsatz in Betracht.
1. Die wichtigste Wirkstoff-Gruppe sind die Aldehyde (Formaldehyd, Glyoxal, Glutaraldehyd). Sie besitzen ein breites Wirkungsspektrum einschließlich Virus-Wirksamkeit und sporizider Wirkung bei Formaldehyd und Glutaraldehyd.
2. Phenol-Derivate besitzen eine gute bakterizide Wirkung, sind aber nicht sporizid. Gegenüber fast allen anderen Desinfektionsmittelwirkstoffen haben sie den Vorzug, durch Schmutz verhältnismäßig wenig beeinflusst zu werden. Sie eignen sich daher bes. zur Stuhldesinfektion. Typische Vertreter sind 2-Biphenylol und p-Chlor-m-kresol (4-Chlor-3-methylphenol).
3. Alkohole zeichnen sich durch schnelle Wirksamkeit aus, allerdings erst bei relativ hohen Konzentrationen von ca. 40-80%.
4. Die quaternären Ammonium-Verbindungen, Kationentenside (Invertseifen) und Amphotenside gehören zur Klasse der Tenside. Sie zeichnen sich durch recht gute Hautund Materialverträglichkeit sowie Geruchsneutralität aus. Ihr Wirkungsspektrum ist dagegen nur begrenzt. Hierher gehören zum Beispiel Benzalkoniumchlorid, Cetrimoniumbromid, Cetylpyridiniumchlorid (Hexadecylpyridiniumchlorid) und andere.
   Quaternäre Ammoniumverbindungen sind organisch Ammoniumverbindungen mit quaternären Stickstoffatomen. Quaternäre Ammoniumverbindungen mit einem hydrophoben Alkyl-Rest sind biozid; ihr Einsatz ist freilich aus toxikologischen Gründen rückläufig. Quaternäre Ammoniumverbindungen werden durch Umsetzung tertiärer Amine mit Alkylierungsmitteln, wie zum Beispiel Methylchlorid, Benzylchlorid, Dimethylsulfat, Dodecylbromid, aber auch Ethylenoxid hergestellt. In Abhängigkeit von dem eingesetzten tert. Amin unterscheidet man drei Gruppen: 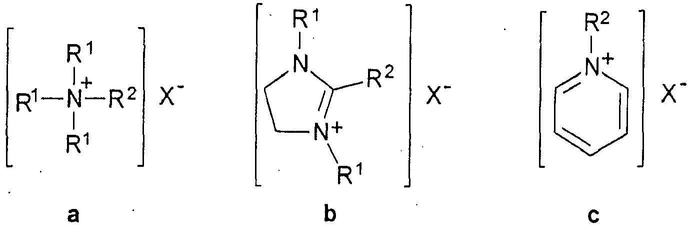
   a) Lineare Alkylammoniumverbindungen
   b) Imidazoliniumverbindungen
   c) Pyridinium-Verb. R¹ = CH₃, R² = C₈₋₁₈, X = Halogen.

   Die Alkylierung tertiärer Amine mit einem langen Alkylrest und zwei Methylgruppen gelingt besonders leicht, auch die Quaternierung tertiärer Amine mit zwei langen Resten und einer Methylgruppe kann mit Hilfe von Methylchlorid unter milden Bedingungen durchgeführt werden. Amine, die über drei lange Alkylreste oder hydroxysubstituierte Alkylreste verfügen, sind wenig reaktiv und werden bevorzugt mit Dimethylsulfat quaterniert.
5. Von den Halogenen besitzen Chlor und Iod eine gewisse Bedeutung als Desinfektionsmittel. Chlor ist von der Wasseraufbereitung und Schwimmbaddesinfizierung her bekannt und damit seine unangenehmen Eigenschaften wie Geruch und Korrosivität. Trotz der ausgezeichneten Wirkung gegen Bakterien, Pilze, Sporen und Viren haben chlorhaltige Desinfektionsmittel im Humanbereich aus den obengenannten Gründen und wegen der starken Chlor-Zehrung durch organ. Substanzen keine starke Verbreitung gefunden. Dagegen werden Hypochlorite, Chlorkalk- und Chlorisocyanursäuren als technische Desinfektionsmittel noch umfänglich benutzt. Iodtinktur wird im medizinischen Bereich als Antiseptikum verwendet.
6. Desinfektionsmittel auf Basis von aktivem Sauerstoff, zum Beispiel Wasserstoffperoxid, Peroxyessigsäure, haben in letzter Zeit wieder etwas an Bedeutung gewonnen.
7. Silber wirkt, auch in gebundener Form, stark antiseptisch, da die in der Oxid-Schicht der Metalloberfläche enthaltenen Ag-Ionen in den Mikroorganismen eine blockierende Wirkung auf die Thiol-Enzyme ausüben. Ag-Ionen wirken auch stark fungizid und bakterizid. Dünne, bakterientötende Silber-Folien werden deshalb als Wundverbandmaterial verwendet, desgleichen Silber-Aerosole, Silber-Lösungen, Silberhaltige Salben, Tabletten und dgl. als Antiseptika und Antimykotika.

Die Silber-Ionen können dabei in Form von Salzen, Zeolithen, z.B. Aluminiumsilikate, oder bevorzugt Silbergläsern eingesetzt werden.

Außer den genannten Mikrobizid-Wirkstoffen sind noch eine Anzahl von mikrobistat. Substanzen und Konservierungsmitteln (Diphenylether, Carbanilide, Acetanilide aromatischen Säuren und deren Salze) für spezifische Verwendung auf dem Markt, die im erweiterten Sinne den Desinfektionsmitteln zugerechnet werden.

Eine einheitliche Wirkungsweise der Desinfektionsmittel ist nicht zu erkennen. Während manche Präparate auf die Cytoplasmamembran der Bakterien zerstörend wirken sollen, wird von anderen eine irreversible Blockierung wichtiger Sulfidbindungen bei Enzymen oder von Spurenelementen durch Chelatisierung angenommen.

Gegenstand der Erfindung ist dementsprechend auch die Verwendung von desinfizierenden Mittel in Polymermatrices, welche
- mindestens ein nichtionisches Tensid und
- mindestens eine Aminosäure und/oder ein Aminosäurenderivat
- sowie mindestens ein desinfizierendes Agens und/oder einen mikrobiziden Wirkstoff enthalten.

Vorteilhaft werden das oder die nichtionische Tenside gewählt aus der Gruppe der Alkylethoxylate und/oder Alkylpropxylate, deren Alkylgruppe eine gesättigte oder ungesättigte, gerad- oder verzweigtkettige Alkylgruppe mit (8) 10 bis 18, vorzugsweise 12 bis 14 Kohlenstoffatomen ist, wobei sie vorzugsweise pro Molekül 2 bis 15, insbesondere 5 bis 9, speziell 7 Ethylenoxideinheiten enthalten. Ganz besonders bevorzugt sind Isotridecanolethoxylat und/oder Fettalkoholpolyglykolether.

Vorteilhaft wird die Gesamtmenge an nichtionischen Tensiden (eine oder mehrere Verbindungen) aus dem Bereich von 1,0 bis 20,0 Gew.-%, vorzugsweise von 5,0 bis 15,0 Gew.-% gewählt, jeweils bezogen auf das Gesamtgewicht der Matrix.

Vorteilhafte Aminosäuren sind zum Beispiel die Glutaminsäure, welche sich durch die folgende Strukturformel auszeichnet: 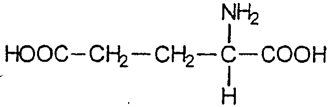 und/oder die Pyrrolidoncarbonsäure (Pyroglutaminsäure), welche sich durch die folgende Strukturformel auszeichnet:

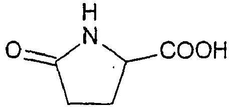

Vorteilhaft wird die Gesamtmenge an Aminosäuren (eine oder mehrere Verbindungen) aus dem Bereich von 0,1 bis 10,0 Gew.-%, vorzugsweise von 0,5 bis 2,0 Gew.-% gewählt, jeweils bezogen auf das Gesamtgewicht der Matrix.

Das oder die desinfizierenden Agenzien (mikrobiziden Wirkstoffe) werden bevorzugt gewählt aus der Gruppe der Aldehyde (zum Beispiel Formaldehyd, Glyoxal, Glutaraldehyd), der Phenol-Derivate (zum Beispiel 2-Biphenylol und p-Chlor-m-kresol (4-Chlor-3-methylphenol), der Alkohole, der quaternären Ammonium-Verbindungen (zum Beispiel Benzalkoniumchlorid, Cetrimoniumbromid, Cetylpyridiniumchlorid (Hexadecylpyridiniumchlorid). Aldehyde und quaternäre Ammoniumverbindungen sind dabei ganz besonders bevorzugt.

In einer besonders vorteilhaften Ausführungsform können die desinfizierenden Systeme ferner Amphotenside enthalten. Amphotenside sind Tenside, die sowohl saure als auch basische hydrophile Gruppen besitzen und sich also je nach Bedingung sauer oder basisch verhalten. Vorteilhaft sind beispielsweise Amphotenside auf der Basis von aliphatischen Polyaminen mit Carboxy-, Sulfo- oder Phosphono-Seitenketten, wie beispielsweise R-NH-(CH₂)ₙ-COOH.

Bevorzugt sind zum Beispiel Amphotenside, deren Alkylgruppe eine gesättigte oder ungesättigte, gerad- oder verzweigtkettige Alkylgruppe mit 10 bis 18, vorzugsweise 12 bis 14 Kohlenstoffatomen ist.

Insbesondere vorteilhaft sind ferner Amphotenside aus der Gruppe der Amphopropionate, wie zum Beispiel das Cocobetainamido Amphopropionat, welches sich durch die folgende Struktur auszeichnet:

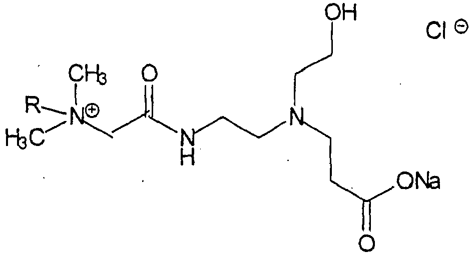

Vorteilhaft wird die Gesamtmenge an Amphotensiden (eine oder mehrere Verbindungen) aus dem Bereich von 1,0 bis 10,0 Gew.-%, vorzugsweise von 2,0 bis 5,0 Gew.-% gewählt, jeweils bezogen auf das Gesamtgewicht der Matrix.

Vorteilhaft ist es, die Verdünnung so durchzuführen, dass der Gehalt der einzelnen Substanzen in der Gebrauchslösung wie folgt ist:

| | |
|---|---|
| nichtionische Tenside: | zwischen 0,005 und 1 Gew.-% |
| Aminosäure: | zwischen 0,0005 und 0,5 Gew.-% |
| gegebenenfalls Amphotenside: | zwischen 0,005 und 0,5 Gew.-% |
| desinfizierende Agenzien: | zwischen 0,01 und 2,0 Gew.-% |

Zusätzlich zu den vorstehend genannten Komponenten können die desinfizierenden Systeme für derartige Zubereitungen übliche Konservierungsstoffe, Farbstoffe, Duftstoffe und/oder andere übliche Hilfsstoffe enthalten. Es ist jedoch auch möglich, solche Komponenten zu verwenden, die eine (konservierende, pflegende usw.) Wirkung entfalten und dabei gleichzeitig für eine bestimmte Farbe und/oder einen angenehmen Duft sorgen.

Die jeweils einzusetzenden Mengen an derartigen Trägerstoffen und Parfüm können in Abhängigkeit von der Art des jeweiligen Produktes vom Fachmann durch einfaches Ausprobieren leicht ermittelt werden.

Vorteilhaft ist auch die Verwendung von desinfizierenden Systemen, welche mindestens einen mikrobiziden Wirkstoff, gewählt aus der Gruppe der Alkylamine mindestens eine Aminosäure und/oder ein Aminosäurenderivat mindestens eine quaternäre Ammoniumverbindung enthalten.

Vorteilhaft werden die quaternären Ammonium-Verbindungen bevorzugt gewählt aus der Gruppe Benzalkoniumchlorid, Didecyldimethylammoniumchlorid, Cetrimoniumbromid, Cetylpyridiniumchlorid (Hexadecylpyridiniumchlorid). Vorteilhaft ist das Alkylamin das Dodecylbispropylentriamin.

Erfindungsgemäß vorteilhaft werden zusätzlich nichtionische Tenside zugesetzt, insbesondere vorteilhaft gewählt aus der Gruppe der Alkylethoxylate, deren Alkylgruppe eine gesättigte oder ungesättigte, gerad- oder verzweigtkettige Alkylgruppe mit 8 bis 18, vorzugsweise 12 bis 14 Kohlenstoffatomen ist, wobei sie vorzugsweise pro Molekül 2 bis 15, insbesondere 5 bis 9, speziell 7 Ethylenoxideinheiten enthalten. Ganz besonders bevorzugt sind Isotridecanolethoxylat und/oder Fettalkoholpolyglykolether.

Vorteilhaft wird die Gesamtmenge an nichtionischen Tensiden (eine oder mehrere Verbindungen) aus dem Bereich von 1,0 bis 20,0 Gew.-%, vorzugsweise von 5,0 bis 15,0 Gew.-% gewählt, jeweils bezogen auf das Gesamtgewicht der Matrix.

Weiterhin sind als Mittel zur Desinfektion, Konservierung und Antiseptik eine Vielzahl mikrobizid wirksamer chemischer Substanzen beziehungsweise Gemische dieser Substanzen an sich bekannt. Mikrobizide Substanzen sind im allgemeinen gegen das übliche Spektrum von Keimen, wie beispielsweise grampositive Bakterien, gramnegative Bakterien, Mykobakterien, Hefen, Pilze, Viren und dergleichen, mehr oder weniger wirksam, so dass man üblicherweise eine ausreichende Desinfektion, Konservierung oder Antiseptik durch geeignete Wirkstoffkombinationen erzielen kann.

Man kennt zur Desinfektion, Konservierung und Antiseptik eine Reihe von Wirkstoffen, insbesondere Aldehyde, wie beispielsweise Formaldehyd oder Glutaraldehyd, quaternäre Ammoniumverbindungen und langkettige Amine, Phenole oder Alkohole,

Aldehyde fixieren Reste von Blut und Eiweiß durch chemische Reaktion an den zu desinfizierenden Gegenständen, so dass diese nach der Desinfektion schwer zu reinigen sind. Außerdem haben sie ein vergleichsweise hohes allergenes Potential, so dass Anwendungen auf Haut und Händen nur in geringen Konzentrationen möglich sind oder aber in Kombination mit weiteren Wirkstoffen in Betracht kommen, um die erforderliche Unterschreitung der Sensibilisierungsschwelle einhalten zu können. Höhere Konzentrationen von Aldehyden sind auch wegen ihrer Geruches unerwünscht, so dass man auch aus diesem Grund die Konzentration durch Kombination mit weiteren Wirkstoffen verringert.

Quaternäre Ammoniumverbindungen und langkettige Amine werden häufig in der Flächendesinfektion und zur manuellen Instrumentendesinfektion sowie in geringem Umfang auch in der Händeantiseptik verwendet. Im Vergleich zu den Aldehyden ist der Geruch dieser Verbindungen deutlich weniger unangenehm. Eine chemische Reaktion mit Eiweißen erfolgt nicht, jedoch kommt es zu einer physikalischen Fällung von Eiweißen, die zum Teil durch geschickte Kombination mit Tensiden kompensiert werden kann. Für die maschinelle Instrumentendesinfektion sind die quaternären Ammoniumverbindungen nicht geeignet, weil es infolge der Turbulenzen in der Reinigungsmaschine zu einer starken, unerwünschten Schaumbildung kommt. Bei der Flächendesinfektion zeigen quaternäre Ammoniumverbindungen eine starke Tendenz, auf den Oberflächen "aufzuziehen", d. h., es bilden sich Schichten dieser Verbindungen auf den Oberflächen aus. Ein weiterer entscheidender Nachteil ist das eingeengte Wirkungsspektrum quaternärer Ammoniumverbindungen, da diese weder sporizid noch gegen unbehüllte Viren wirken. Phenole sind vor allem wegen ihres Geruches, ihrer geringen Wirksamkeit gegen den Poliovirus, ihrer zum Teil schlechten Abbaubarkeit, ihrer hohen Lipidlöslichkeit verbunden mit einer starken Penetration durch die Haut sowie toxischer und mutagener Risiken in nahezu allen Anwendungsbereichen für Desinfektionsmittel auf dem Rückzug.

Die aliphatischen Alkohole Ethanol, Propanol-1 und Propanol-2 sind als Wirkstoffe zur Desinfektion von Haut und Händen beziehungsweise für die Haut- und Händeantiseptik seit langem bekannt. Mit Desinfektionsmitteln und Antiseptika auf der Basis von Alkoholen können bei kurzen Einwirkzeiten von 30 bis 60 Sekunden Keimzahlreduktionen von bis zu 99,9 % erzielt werden. Eine allgemeine, kurzgefasste Darstellung der mikrobiziden Wirksamkeit von Alkoholen findet sich in dem Buch: K.H. Wallhäußer, "Praxis der Sterilisation, Desinfektion und Konservierung", G. Thieme Verlag, Stuttgart, New York, 5. Auflage, S. 469 - 474*.*

Alkohole besitzen eine bakterizide Wirkung, die von Methanol zu Propanol zunimmt. Verwendet werden vor allem Ethanol, n-Propanol und Isopropanol, wobei der Alkoholgehalt der Zubereitungen im allgemeinen zwischen 50 und 80 % liegt. Der wesentliche Vorteil von Alkoholen ist, dass der Wirkungseintritt sehr rasch erfolgt. Nachteilig ist, dass sie nicht gegen Sporen wirksam sind und dass die Wirkung nach sehr kurzer Zeit endet, da Alkohole schnell verdunsten. Eine antivirale Wirksamkeit von Alkoholen wird zwar diskutiert, aber erst jenseits einer hohen Konzentrationsgrenze, welche bei Ethanol bei ca. 80 % vermutet wird.

Es hat sich in der Praxis gezeigt, dass alkoholische Desinfektionsmittel und Antiseptika Viren und Spuren von Bacillus- und Clostridienarten nicht oder nicht in hinreichendem Maße abzutöten vermögen. Zwar kann man die Sporenfreiheit von alkoholischen Lösungen durch Filtration erreichen, allerdings kann in der Praxis nicht vollständig ausgeschlossen werden, dass Keimsporen (nachträglich) in die Präparate gelangen, beispielsweise beim kurzzeitigen Öffnen der Aufbewahrungsgefäße oder beim Abfüllen der Mittel in Behälter, die bereits Sporen enthalten. Aus diesem Grund besteht bei der Verwendung von alkoholischen Haut- und Handantiseptika stets ein gewisses Risiko einer durch Sporen verursachten Infektion.

Antiseptika sind besonders geeignet zur Behandlung der Haut. Antiseptika zeigen eine sehr gute Wirksamkeit gegen Dermatophyten und zeichnen sich überraschenderweise insbesondere dadurch aus, dass sie eine gute Wirksamkeit gegen Viren haben.

Die Bestandteile der Antiseptika agieren in bezug auf ihre antimikrobiellen und antiviralen Eigenschaften synergistisch, also in signifikanter Weise überadditiv.

Vorteilhaft ist demgemäss auch die Verwendung einer Zubereitung aus

| | |
|---|---|
| (a) 42 - 47 Gew.-% | 1-Propanol |
| (b) 22 - 27 Gew.-% | 2-Propanol |
| (c) 4 - 6 Gew.-% | Ethanol |
| (d) Mindestens 20 Gew.-% | Wasser |
| (e) Höchstens 0,0001 Gew.-% | an Substanzen, welche unter Normalbedingen als Festkörper vorliegen |
| (f) Keinen wirksamen Gehalt | an weiteren Substanzen, welche sich durch viruzide Eigenschaften auszeichnen |

als Antiseptikum, insbesondere die Verwendung zur Bekämpfung oder Inaktivierung des HIV-Virus oder des Hepatitis B-Virus.

Besonders geeignet als Antiseptikum ist wiederum Chlorhexidin, 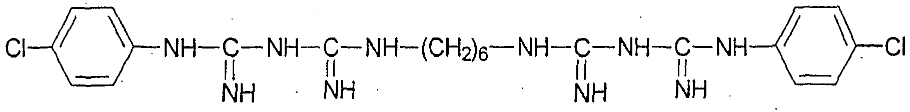 internationaler Freiname für 1,1'-Hexamethylenbis[5-(4-chlorphenyl)-biguanid], wobei als Antiseptikum das Dihydrochlorid, Diacetat und Digluconat verwendet werden.

Zur Anwendung als Pflaster bzw. kosmetische Matrix / kosmetisches Pad werden die erfindungsgemäßen Gelmatrices als Schicht auf ein Trennmedium aus Papier, Folie o. ä. gepresst, gewalzt o. ä. und auf der Rückseite mit einem beliebigen Trägermaterial wie z.B. einer Polymerfolie, Textilien o.ä. kaschiert. Erfindungsgemäß besonders bevorzugt werden die Gelmatrices im warmen Zustand mittels Dosierpumpe auf ein Trägermaterial aufgetragen und ganz besonders bevorzugt durch entsprechende Kavitäten in den Press- oder Walzwerken in einer dreidimensionalen Form ausgeführt. Die Form der erzeugten Pflaster bzw, kosmetischen Matrix wird durch die Form der Kavitäten bestimmt und unterliegt keiner Einschränkung, sie kann z.B. ellipsoid mit flach auslaufenden Rändern oder beispielsweise eckig ausgeführt sein.
Besonders vorteilhaft ist die erfindungsgemäße Gelmatrix auf einer flexiblen Deckschicht aufgebracht, insbesondere bei der Verwendung als Pflaster / kosmetische Matrix. Aufgebaut ist ein entsprechendes Pflaster / eine entsprechende kosmetische Matrix aus einem Träger wie Folien, Vliese, Gewebe, Schäume etc., der Klebmatrix und Abdeckfolie, Abdeckpapier oder Trennpapier zum Schutz der klebenden Matrix vor dem Gebrauch des Pflasters / der kosmetischen Matrix.
In einer weiteren bevorzugten Ausführungsform der Erfindung werden als Träger Polymerfolien, Vliese, Gewebe sowie deren Kombinationen eingesetzt. Als Trägermaterialien stehen u.a. Polymere wie Polyethylen, Polypropylen, Polyester, Polyether, Polyether-ester Copolymere und Polyurethan oder auch Naturfasern zur Auswahl.
Zusammenfassend kann festgehalten werden, dass als Trägermaterialien sich alle starren und elastischen Flächengebilde aus synthetischen und natürlichen Rohstoffen eignen. Bevorzugt sind Trägermaterialien, die so eingesetzt werden können, dass sie Eigenschaften eines funktionsgerechten Verbandes erfüllen. Beispielhaft sind Textilien wie Gewebe, Gewirke, Gelege, Vliese, Laminate, Netze, Folien, Schäume und Papiere aufgeführt. Weiter können diese Materialien vor- beziehungsweise nachbehandelt werden. Gängige Vorbehandlungen sind Corona und Hydrophobieren; geläufige Nachbehandlungen sind Kalandern, Tempern, Kaschieren, Stanzen und Eindecken. Besonders vorteilhaft ist, wenn das Trägermaterial sterilisierbar, bevorzugte γ-(gamma) sterilisierbar, ist.

Erfindungsgemäß ganz besonders bevorzugt sind Trägermaterialien mit einer guten Sauerstoff-, Luft- und Wasserdampfdurchlässigkeit, welche im Siebdruck oder analogen Verfahren punktuell mit stark klebenden Polymeren wie Polyisobutylen, SEBS-Blockpolymeren, Natur- und/oder Synthesekautschuken, Polyurethan o. ä. versehen sind und an den Seitenrändern die aufgebrachte Hydrogelmatrix nach außen überlappen. Dergestalt ausgefertigte erfindungsgemäße Matrices können an mechanisch stark beanspruchten Körperteilen wie Ellenbogen oder Kniegelenken selbstklebend fixiert werden, wo das eigene Haftvermögen der Hydrogele/Cataplasmen für eine dauerhafte Applikation nicht mehr genügt.

Die genannten Eigenschaften der Klebmatrix legen insbesondere die Verwendung für medizinische Produkte, insbesondere Pflaster, medizinische Fixierungen, Wundabdeckungen, orthopädische oder phlebologische Bandagen und Binden nahe. Ganz besonders bevorzugt ist die Verwendung als kosmetisches oder dermatologisches Pad.

Schließlich kann die Gelmatrix mit einem klebstoffabweisenden Trägermaterial, wie silikonisiertes Papier, eingedeckt oder mit einer Wundauflage oder einer Polsterung versehen werden. Auf seiner selbstklebend ausgerüsteten, später der Haut zugewandten Seite ist die erfindungsgemäße kosmetische Matrix über seine ganze Breite bis zum Gebrauch üblicherweise mit einem klebstoffabweisenden Trägermaterial abgedeckt. Dieses schützt die Selbstklebeschicht aus der gut hautverträglichen Klebemasse der Gelmatrix, die vorzugsweise im Transferverfahren aufgebracht worden ist, und stabilisiert zusätzlich das ganze Produkt. Die Abdeckung kann in bekannter Weise einstückig oder vorzugsweise zweiteilig ausgebildet sein.
Weitere Ausführungsformen können dergestalt sein, dass zwischen der Rückseite der Matrix und dem Abdeckträger sich eine zweite Matrix mit höherer Wirkstofflöslichkeit als Reservoir befindet. Dies könnte statt einer zweiten Matrix und Träger auch eine Tiefziehfolie mit reinem Wirkstoff sein.
Auf der Klebseite der Matrix befindet sich teilweise, z.B. am Rand, eine zweite Matrix mit hoher Klebkraft zur zusätzlichen Fixierung, aber ungenügender Wirkstofflöslichkeit.
Die wirkstofffreie Matrix befindet sich zwischen zwei nicht verankernden Folien und wird zur Fixierung genutzt.

Gegenstand der vorliegenden Erfindung ist ferner die Verwendung kosmetischer oder dermatologischer Wirkstoffe in den erfindungsgemäßen Gelmatrices.
Insbesondere die Verwendung der wirkstoffdotierten Gelmatrices auf Polyacrylsäure-Agar-Agar-Basis zur Anwendung als PADs zur kosmetischen und wohltuenden Behandlung von unerwünschten Hauterscheinungen ist bevorzugt hervorzuheben. Insbesondere bei der Behandlung von Hautalterungserscheinungen, insbesondere Hautfalten, bei Pigmentierungsstörungen, insbesondere von Altersflecken, und entzündlichen/irritativen Hauterscheinungen, beispielsweisebei Rasurbrand und/oder Sonnenbrand.

Es ist bevorzugt im Sinne der vorliegenden Erfindung, wenn die erfindungsgemäßen kosmetischen oder dermatologischen Matrices zusätzlich einen oder mehrere Alkohole enthalten, insbesondere, wenn die Formulierungen in Form eines Aftersun-Präparats vorliegen und sich durch eine besondere Kühlwirkung auszeichnen sollen.

Die Verwendung der Polymermatrix als kosmetische oder dermatologische Pads oder Pflaster ist besonders in flächiger Ausführungsform mit einer Gesamtfläche von 0,2 bis 1000 cm² geeignet. Damit werden beispielweise kleine (0,2 - 2 cm²) Bereiche der Haut oder großflächige Bereiche (bis zu 1000 cm²) zur intensiven Kühlung abgedeckt.

Bevorzugt ist die Verwendung der selbstklebenden Polymermatrix in flächiger oder räumlicher Ausführungsform mit einem Polymermatrixgewichtsanteil von 0,1 bis 1000 g, insbesondere von 500 g. Die Form kann dabei rund, oval, eckig oder den Hautpartien angepasst gestaltet sein.

Die nachfolgenden Beispiele veranschaulichen die Erfindung ohne sie einzuschränken. In den nachfolgenden Tabellen sind erfindungsgemäße Gelmatrices aufgeführt. Die angegebenen Massenanteile beziehen sich auf die Gesamtmasse der Matrix.

### Beispiele I - III

| Bestandteil | **I** | **II** | **III** |
|---|---|---|---|
| Wasser | ad 100 % | ad 100 % | ad 100 % |
| Agar-Agar | 2,0 % | 2.0 % | 2,0 % |
| Glycerin | 20,0 % | 30,0 % | 40,0 % |
| Süßholzextrakt | 0,1% | 0,1 % | 0,1 % |
| Carbopol 980 | 8,0 % | 8,0 % | 8,0 % |
| NaOH | 0,1 % | 0,1 % | 0,1 % |

Die Beispiele I - III zeigen bei konstantem Gehalt an Polyacrylsäure analoges Haftvermögen und bei ebenfalls konstantem Gehalt an Agar-Agar aber steigendem Gehalt an Glycerin zunehmende Kohäsivität/Elastizität.

### Beispiele IV - VI

| Bestandteil | **IV** | **V** | **VI** |
|---|---|---|---|
| Wasser | ad 100 % | ad 100 % | ad 100 % |
| Agar-Agar | 2,0 % | 2,0 % | 2,0 % |
| Liponamid | 0,1 % | 0.05 % | 0,1 % |
| Glycerin | 30,0 % | 30,0 % | 30,0 % |
| Carbopol 980 | 8,0 % | 12,0 % | 16,0 % |
| NaOH | 0,1 % | 0,1 % | 0,1 % |

Die Beispiele IV - VI zeigen bei konstantem Gehalt an Agar-Agar und Glycerin analoge Kohäsivität/Elastizität und bei gleichzeitig steigendem Gehalt an Polyacrylsäure zunehmendes Haftvermögen.

### Beispiele VII - IX

| Bestandteil | **VII** | **VIII** | **IX** |
|---|---|---|---|
| Wasser | ad 100 % | ad 100 % | ad 100 % |
| Sorbitol | 15,7 % | 15,0 % | 15,7 % |
| Agar-Agar | 2,0 % | 2,0 % | 2,0 % |
| Glycerin | 15,0 % | 15,0 % | 15,0 % |
| Carbopol 980 | 8,0 % | 8,0 % | 8,0 % |
| Kreatin | 0,2 % | 0,25 % | 0.1 % |
| NaOH | 0,1 % | 0,1 % | 0,1 % |
| Propandiol | 5,0 % | ----- | ----- |
| Menthol | 1,0 % | ----- | ----- |
| Dexpanthenol | 1,0 % | ----- | ----- |
| Capsicum-Extrakt | ----- | 3,0 % | ----- |
| Chlorhexidindigluconat | ----- | ----- | 1,0 % |

Die erfindungsgemäßen Formulierungen sind in jeglicher Hinsicht überaus befriedigende Präparate, die sich durch eine hervorragende Wirkung auszeichnen. Bei Anwendung der erfindungsgemäß verwendeten Wirkstoffe bzw. kosmetischer oder topischer dermatologischer Zubereitungen mit einem wirksamen Gehalt an erfindungsgemäß verwendeten Wirkstoffen ist eine wirksame Behandlung, aber auch eine Prophylaxe von entzündlichen Hautzuständen - auch dem atopischen Ekzem - und/oder zum Hautschutz bei empfindlich determinierter trockener Haut möglich. Der erfindungsgemäße Wirkstoff bzw. kosmetische oder topische dermatologische Zubereitungen mit einem wirksamen Gehalt an erfindungsgemäßem Wirkstoff dient aber auch in überraschender Weise zur Beruhigung von empfindlicher oder gereizter Haut.

Als besonders geeignet für die Anwendung bei Altersflecken haben sich folgende Zubereitungen erweisen

### Beispiele X-XII

| Bestandteil | **X** | **XI** | **XII** |
|---|---|---|---|
| Wasser | ad 100 % | ad 100 % | ad 100 % |
| Sorbitol | 15,7 % | 15,7 % | 15,7 % |
| Agar-Agar | 2,0 % | 2,0% | 2,0 % |
| Propandiol | 5,0% | 5,0 % | 5,0 % |
| Glycerin | 15,0% | 15,0% | 15,0% |
| Carbopol 980 | 8,0% | 8,0% | 8,0% |
| NaOH | 0.1 % | 0,1 % | 0,1 % |
| Kreatin | ---- | ---- | 0,5% |
| Liponamid | 0,05 % | 0.1% | ----- |

Als besonders geeignet für die Anwendung bei enzündlichen und irritativen Hautzuständen, insbesondere nach Rasur- und Sonnenbrand haben sich folgende Zubereitungen erweisen:

### Beispiele XIII-XV

| Bestandteil | **XIII** | **XIV** | **XV** |
|---|---|---|---|
| Wasser | ad 100 % | ad 100 % | ad 100 % |
| Sorbitol | 15,7 % | 15,7 % | 15,7 % |
| Agar-Agar | 2,0 % | 2,0 % | 2,0 % |
| Propandiol | 5,0 % | 5,0 % | 5,0 % |
| Glycerin | 15,0% | 15,0% | 15,0% |
| Carbopol 980 | 8,0% | 8,0% | 8,0% |
| NaOH | 0,1 % | 0,1 % | 0,1 % |
| Süßholzextrakt | 0,01% | 0,05% | --- |

Als besonders geeignet für die Anwendung bei Altershautfalten haben sich folgende Zubereitungen erweisen:

### Beispiele XVI-XVIII

| Bestandteil | **XVI** | **XVII** | **XVIII** |
|---|---|---|---|
| Wasser | ad 100 % | ad 100 % | ad 100 % |
| Sorbitol | 15,7 % | 15,7 % | 15,7 % |
| Agar-Agar | 2.0 % | 2,0 % | 2,0 % |
| Propandiol | 5,0 % | 5,0% | 5,0 % |
| Glycerin | 15,0% | 15,0% | 15,0% |
| Carbopol 980 | 8,0% | 8,0% | 8,0% |
| NaOH | 0,1 % | 0,1 % | 0,1 % |
| Q10 | ---- | ---- | 0,1% |
| Kreatin | 0,5 % | 0,1% | ----- |

## Patentansprüche

1. Selbstklebende Polymermatrix gebildet aus einem in Wasser gelbildendem Polymer, Wasser, einem mit Alkohol nicht gelbildendem Meeresalgenextrakt und ein- oder mehrwertigen Alkohol, **dadurch gekennzeichnet, dass** das in Wasser gelbildende Polymer aus mindestens einem Polyacrylsäurepolymer besteht und der Alkohol aus Glycerin oder Propandiol gewählt wird.

2. Polymermatrix nach Anspruch 1, **dadurch gekennzeichnet, dass** als Polyacrylsäurepolymere Acrylat-Alkylacrylat-Copolymere mit folgender Struktur ausgewählt werden: 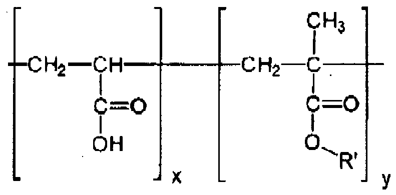 wobei R' ein Alkylrest und x bzw. y den jeweilige stöchiometrische Anteil der jeweiligen Comonomere darstellt.

3. Polymermatrix nach Anspruch 2 oder 3, **dadurch gekennzeichnet, dass** als Polyacrylsäurepolymere Copolymere aus C₁₀₋₃₀-Alkylacrylaten und einem oder mehreren Monomeren der Acrylsäure, der Methacrylsäure oder deren Ester, die kreuzvernetzt sind mit einem Allylether der Saccharose oder einem Allylether des Pentaerythrit, verwendet werden.

4. Polymermatrix nach einem der vorstehenden Ansprüche, **dadurch gekennzeichnet, dass** das in Wasser gelbildende Polymer in einer Menge von 2 - 55 Gew. %, bezogen auf die Gesamtmasse der Matrix, eingesetzt wird.

5. Polymermatrix nach Anspruch 4, **dadurch gekennzeichnet, dass** das in Wasser gelbildende Polymer in einer Menge von 5 - 30 Gew.% eingesetzt wird.

6. Polymermatrix nach einem der vorstehenden Ansprüche, **dadurch gekennzeichnet, dass** als Meeresalgenextrakt Agar-Agar und/oder Carrageenan gewählt wird.

7. Polymermatrix nach einem der vorstehenden Ansprüche, **dadurch gekennzeichnet, dass** der Meeresalgenextrakt in einer Menge von 0,1 - 15 Gew.%, bezogen auf die Gesamtmasse der Matrix, eingesetzt wird.

8. Polymermatrix nach Anspruch 7, **dadurch gekennzeichnet, dass** der Meeresalgenextrakt in einer Menge von 0,5 - 5 Gew.% eingesetzt wird.

9. Polymermatrix nach einem der vorstehenden Ansprüche, **dadurch gekennzeichnet, dass** der Alkohol in einer Menge von 1 - 85 Gew.%, bezogen auf die Gesamtmasse der Matrix, eingesetzt wird.

10. Polymermatrix nach Anspruch 9, **dadurch gekennzeichnet, dass** der Alkohol in einer Menge von 5 - 45 Gew.%, bezogen auf die Gesamtmasse der Matrix, eingesetzt wird.

11. Polymermatrix nach einem der vorstehenden Ansprüche, **dadurch gekennzeichnet, dass** zusätzlich mindestens ein Wirkstoff enthalten ist.

12. Polymermatrix nach Anspruch 11, **dadurch gekennzeichnet, dass** der Wirkstoff gewählt wird aus der Gruppe Kreatin/Kreatinin, alphaGlucosylrutin, Taurin, Serinol/Isoserinol, Licorice Aqua PU, Licorice PU, Silymarin/Silyphos, Liponsäure, Liponamid, Grüntee-Extrakt, Vitamin C, 8-Hexadecen-1,16-Dicarbonsäure, Isoflavon, Isoflavonoid-haltige Pflanzenextrakte, wie Soja und Klee-Extrakte, Ubichinon Q10, Sericoside, Tyrosinsulfat, Jojobaöl, Aloe vera, Desinfektionsmittel und/oder Antiseptika.

13. Polymermatrix nach Anspruch 11 oder 12, **dadurch gekennzeichnet, dass** der oder die Wirkstoffe in Mengen von 0 - 35 Gew.%, bevorzugt 0,02 - 2% Gew.% enthalten sind.

14. Medizinsche Pflaster, kosmetische oder dermatologische Matrices oder Pads enthaltend eine Polymermatrix nach einem der vorstehenden Ansprüche.

15. Verwendung der selbstklebenden Polymermatrix nach einem der vorstehenden Ansprüche 1 bis 13 in kosmetischen oder dermatologischen Pads oder Pflastern.

16. Nicht-therapeutische Verwendung der selbstklebenden Polymermatrix nach einem der vorstehenden Ansprüche 1 bis 13 zur allgemeinen kosmetischen Hautpflege.

17. Verwendung der selbstklebenden Polymermatrix nach einem der Ansprüche 15 bis 16 in flächiger Ausführungsform mit einer Gesamtfläche von 0,2 bis 1000 cm².

18. Verwendung der selbstklebenden Polymermatrix nach einem der Ansprüche 15 bis 17 in einer Ausführungsform mit einem Matrixanteil von 0,1 bis 1000 g, insbesondere 500g.

## Claims

1. Self-adhesive polymer matrix formed from a polymer which forms gel in water, water, a seaweed extract which does not form gel with alcohol, and monohydric or polyhydric alcohol **characterized in that** the polymer which forms gel in water is composed of at least one polyacrylic acid polymer and the alcohol is selected from glycerin or propanediol.

2. Polymer matrix according to Claim 1, **characterized in that** polyacrylic acid polymers selected are acrylate-alkyl acrylate copolymers having the following structure: 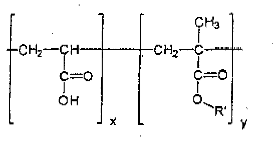 where R' is an alkyl radical and x and y respectively denote the respective stoichiometric fraction of the respective comonomers.

3. Polymer matrix according to Claim 2 or 3, **characterized in that** polyacrylic acid polymers used are copolymers of C₁₀₋₃₀ alkyl acrylates and one or more monomers of acrylic acid, methacrylic acid or esters thereof which are crosslinked with an allyl ether of sucrose or with an allyl ether of pentaerythritol.

4. Polymer matrix according to any one of the preceding claims, **characterized in that** the polymer which forms gel in water is used in an amount of 2%-55% by weight, based on the total mass of the matrix.

5. Polymer matrix according to Claim 4, **characterized in that** the polymer which forms gel in water is used in an amount of 5%-30% by weight.

6. Polymer matrix according to any ona of the preceding claims, **characterized in that** agar-agar and/or carrageenan are/is chosen as seaweed extract.

7. Polymer matrix according to any one of the preceding claims, **characterized in that** the seaweed extract is used in an amount of 0.1%-15% by weight, based on the total mass of the matrix.

8. Polymer matrix according to Claim 7, **characterized in that** the seaweed extract is used in an amount of C.5%-5% by weight.

9. Polymer matrix according to any one of the preceding claims, **characterized in that** the alcohol is used in an amount of 1%-85% by weight, based on the total mass of the matrix.

10. Polymer matrix according to Claim 9, **characterized in that** the alcohol is used in an amount of 5%-45% by weight, based on the total mass of the matrix.

11. Polymer matrix according to any one of the preceding claims, **characterized in that** additionally at least one active substance is present.

12. Polymer matrix according to Claim 11, **characterized in that** the active substance is chosen from the group consisting of creatine/creatinine, alpha-glucosylrutin, taurine, serinol/isoserinol, liquorice aqua PU, Liquorice PU, silymarin/silyphos, lipoic acid, liponamide, green tea extract, vitamin C, 8-hexadecene-1,16-dicarboxylic acid, isoflavone, isoflavonoid-containing plant extracts, such as soya and clover extracts, ubiquinone Q10, sericosides, tyrosine sulfate, jojoba oil, aloe vera, disinfectants and/or antiseptics.

13. polymer matrix according to Claim 11 or 12, **characterized in that** the active substance or substances is or are present in amounts of 0-35% by weight, preferably 0.02-2% by weight.

14. Medical patches, cosmetic or dermatological matrices or pads comprising a polymer matrix according to any one of the preceding claims.

15. Use of the self-adhesive polymer matrix according to any one of the preceding Claims 1 to 13 in cosmetic or dermatological pads or plasters.

16. Non-therapeutic use of the self-adhesive polymer matrix according to any one of the preceding Claims 1 to 13 for general cosmetic skincare.

17. Use of the self-adhesive polymer matrix according to either of Claims 15 and 16 in two-dimensional embodiment with a total area of 0.2 to 1000 cm².

18. Use of the self-adhesive polymer matrix according to any one of Claims 15 to 17 in an embodiment with a matrix fraction of 0.1 to 1000 g, in particular of 500 g.

## Revendications

1. Matrice polymère autoadhésive formée d'un polymère formant un gel dans l'eau, d'eau, d'un extrait d'algue marine ne formant pas de gel avec un alcool et d'un alcool monovalent ou polyvalent, **caractérisée en ce que** le polymère formant un gel dans l'eau est constitué par au moins un polymère de poly(acide acrylique) et l'alcool est choisi parmi le glycérol ou le propanediol.

2. Matrice polymère selon la revendication 1, **caractérisée en ce qu'**on choisit comme polymères de poly(acide acrylique) des copolymères d'acrylate-acrylate d'alkyle présentant la structure suivante : 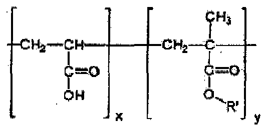 où R' représente un radical alkyle et x et y la proportion stoechiométrique respective des différents comonomères.

3. Matrice polymère selon la revendication 2 ou 3, **caractérisée en ce qu'**on utilise comme polymères de poly(acide acrylique) des copolymères d'acrylates d'alkyle en C₁₀₋₃₀ et d'un ou de plusieurs monomères d'acide acrylique, méthacrylique ou leurs esters, qui sont réticulés avec un allyléther du saccharose ou un allyléther du pentaérythritol.

4. Matrice polymère selon l'une quelconque des revendications précédentes, **caractérisée en ce que** le polymère formant un gel dans l'eau est utilisé en une quantité de 2-55% en poids par rapport à la masse totale de la matrice.

5. Matrice polymère selon la revendication 4, **caractérisé en ce que** le polymère formant un gel dans l'eau est utilisé en une quantité de 5-30% en poids.

6. Matrice polymère selon l'une quelconque des revendications précédentes, **caractérisée en ce qu'**on utilise comme extrait d'algue marine de l'agar-agar et/ou du carraghénane.

7. Matrice polymère selon l'une quelconque des revendications précédentes, **caractérisée en ce que** l'extrait d'algue marine est utilisé en une quantité de 0,1-15% en poids par rapport à la masse totale de la matrice.

8. Matrice polymère selon la revendication 7, **caractérisée en ce que** l'extrait d'algue marine est utilisé en une quantité de 0,5-5% en poids.

9. Matrice polymère selon l'une quelconque des revendications précédentes, **caractérisée en ce que** l'alcool est utilisé en une quantité de 1-85% en poids par rapport à la masse totale de la matrice.

10. Matrice polymère selon la revendication 9, **caractérisée en ce que** l'alcool est utilisé en une quantité de 5-45% en poids par rapport à la masse totale de la matrice.

11. Matrice polymère selon l'une quelconque des revendications précédentes, **caractérisée en ce qu'**au moins une substance active est en outre contenue.

12. Matrice polymère selon la revendication 11, **caractérisée en ce que** la substance active est choisie dans le groupe créatine/créatinine, alpha-glucosylrutine, taurine, sérinol/isosérinol, Licorice Aqua PU, Licorice PU, silymarine/silyphos, acide liponique, liponamide, extrait de thé vert, vitamine C, acide B-hexadécène-1,16-dicarboxylique, isoflavone, extraits de plantes contenant des isoflavonoïdes, tels que des extraits de soja et de trèfle, ubiquinone Q10, séricoside, sulfate de tyrosine, huile de jojoba, Aloe vera, agents de désinfection et/ou antiseptiques.

13. Matrice polymère selon la revendication 11 ou 12, **caractérisée en ce que** la ou les substances actives sont contenues en des quantités de 0-35% en poids, de préférence de 0,02-2% en poids.

14. Pansements médicaux, matrices ou compresses cosmétiques ou dermatologiques contenant une matrice polymère selon l'une quelconque des revendications précédentes.

15. Utilisation de la matrice polymère autoadhésive selon l'une quelconque des revendications précédentes 1 à 13 dans des compresses ou des pansements cosmétiques ou dermatologiques.

16. Utilisation non thérapeutique de la matrice polymère autoadhésive selon l'une quelconque des revendications précédentes 1 à 13 pour les soins cosmétiques généraux.

17. Utilisation de la matrice polymère autoadhésive selon l'une quelconque des revendications 15 à 16 dans une forme de réalisation plate présentant une surface totale de 0,2 à 1000 cm².

18. Utilisation de la matrice polymère autoadhésive selon l'une quelconque des revendications 15 à 17 dans une forme de réalisation présentant une proportion de matrice de 0,1 à 1000 g, en particulier de 500 g.
